# EUROPEAN PATENT SPECIFICATION

(11) **EP 1 253 903 B1**
(45) Date of publication and mention of the grant of the patent: **17.05.2006**
(21) Application number: 01905275.2
(22) Date of filing: 31.01.2001
(51) Int. Cl.: A61K 8/00, A61K 9/00, A61K 9/26

(54) **SURFACTANT FREE TOPICAL COMPOSITIONS AND METHOD FOR RAPID PREPARATION THEREOF**
TENSIDFREIETOPISCHE ZUSAMMENSETZUNGEN UND VERFAHREN ZUR SCHNELLE HERSTELLUNG DAVON
COMPOSITIONS TOPIQUES NE CONTENANT PAS DE TENSIOACTIF ET PROCEDE DE PREPARATION RAPIDE DE CES DERNIERES

(30) Priority: 31.01.2000 US 179057 P; 23.03.2000 US 191878 P; 07.07.2000 US 216847 P
(43) Date of publication of application: 06.11.2002
(73) Proprietor: ENGELHARD CORPORATION, Iselin, NJ 08830-0770 (US)
(72) Inventor: WILMOTT, James, M., Shoreham, NY 11786 (US); AUST, Duncan, T., Carson City, Nevada 89703 (US); CRAWFORD, Timothy, K., Manchester, IA 52057 (US); HAYWARD, James, A., Stony Brook, NY 11790 (US)
(74) Representative: Olsen, Lars Pallisgaard
(86) International application number: PCT/US2001/003157
(87) International publication number: WO 2001/054663

(56) References cited:
- WO-A-00/66085
- WO-A-98/10746
- US-A- 4 927 687

## Description

### FIELD OF THE INVENTION

The present invention relates to surfactant free topical compositions and a rapid method for the preparation of the same.

### BACKGROUND OF THE INVENTION

Most topical preparations currently sold contain a wide variety of physiologically active agents and/or aesthetic modifying agents. Physiologically active agents are compounds which cause a physical change to the body following their application. Examples of such agents include alpha hydroxy acids, antioxidants, and vitamins. Aesthetic modifying agents provide the composition with a defined physical characteristic such as, for example, the degree of moisturization, oil content, and physical form of the composition. Some examples of aesthetic modifying agents include silicone fluids and derivatives, waxes, botanical (vegetable) oils, hydrocarbon-based oils, esters and fragrances.

The performance of these active agents is dependent upon the vehicle used to deliver them. These vehicles range from simple solvents, such as water and ethanol, to complex emulsions. Unfortunately not all active agents are completely soluble or compatible with all vehicles. For example, oil soluble active agents are typically not compatible with water or water-based gel vehicles. As a result, many are thermodynamically unstable and result in a commercially unacceptable shelf life. WO9810746 describes a dermatological composition based on a semi-solid gel carrier system in which microparticulate pharmaceutical drug precipitates are formed in ajustable ratio to dissolved pharmaceutical. EP 0392426A2 describes an emulsifier-free hand and body lotion comprising a mixture of a gelled water system, a particulate carrier material of a hydrophobic polymer and an active ingredient dispersed uniformly throughout and entrapped within the polymer.

Topical preparations having a non-water based solvent are typically not cosmetically elegant, *i.e.,* they do not have an aesthetically pleasing appearance, feel, and/or fragrance. Furthermore, non-water based solvents can cause unwanted side effects, such as irritation or damage to the epithelial surface to which they are applied.

To avoid the problems associated with water and non-water based solvents, stable emulsions are commonly employed to deliver physiologically active agents and aesthetic modifying agents. These emulsions form either spherical micelles of one or more hydrophobic liquid materials in water or spherical droplets of water in a hydrophobic fluid. Such emulsions are typically formed by separately preparing an oil phase and a water phase and mixing the two phases together. In other words, the hydrophobic ingredients are dissolved in a suitable oil phase and the hydrophilic ingredients are dissolved in water. The two phases are then combined with one or more emulsifying agents which are incorporated into either or both the water and oil phases. The emulsifying agents reduce the surface tension between the oil and water phases, thereby making the combination of the two phases more stable.

Such emulsions are generally prepared by heating the oil and water phases to a temperature of 70° C or greater before combining them. The oil and water phases are combined and then slowly cooled to ensure the formation of crystalline structures which enhance the stability of the emulsion. These emulsions usually have a homogeneous opaque white appearance and a smooth or pleasant feeling upon application to the skin or other epithelial surface. However, the use of such emulsions to delivery physiological and/or aesthetic benefits has many limitations.

The presence of significant amounts of surfactant can strip the material lipid barrier of the skin or the lipid bilayer of epithelial cell membranes leaving the tissue vulnerable. Thus, the surfactants themselves can evoke an irritation. Furthermore, the damaged barrier permits the passage of other materials that can cause irritation or increase skin sensitivity and allergic reactions. The literature is replete with clinical evidence of the damaging consequences that can occur with the use or overuse of surfactants. For example, Effendy I., Maibach H.I, "Surfactants and experimental irritant contact dermatitis", *Contact Dermatitis,* 33(4);217-25 (10/1995), indicates that "[m]any surfactants elicit irritant reactions when applied to the skin, partially due to their relative ability to solubilize lipid membranes." Barany E., Lindberg M., Loden M., "Biophysical characterization of skin damage and recovery after exposure to different surfactants", *Contact Dermatitis* 40(2):98-103 (2/1999), states that "[t]he majority of adverse skin reactions to personal care products are presumed to be caused by irritant substances, like surfactants."

Moreover, there are limitations to conventional topical preparations. For example, many materials having aesthetic properties are not easily incorporated into an emulsion, such as, for example, fluorinated compounds. Additionally, each time the oil or water phase is changed in a formulation, the amount and type of emulsifying agents in the formulation needs to be readjusted.

Many topical preparations formulated contain active agents and/or aesthetic modifying agents which readily become destabilized in emulsions, causing them to degenerate and/or deteriorate. For example, prolonged heating of the water and oil phases can thermodynamically modify the active agent or can kinetically accelerate the reaction of the active agent with another agent in the emulsion or with air if the material is oxygen sensitive.

Moreover, lowering the surface tension of a topical preparation generally increases the surface exposure of the active agent or aesthetic modifying agent to oxygen and other destabilizing materials. For example, in a topical preparation containing retinol as an active ingredient, the instability of the preparation may decrease the efficacy of the retinol. The instability of an unsaturated fatty acid as an aesthetic modifying agent leads to color changes in the preparation and malodor.

Since the time between manufacturing and sale of a cosmetic product is typically several weeks, the product is often no longer "fresh" or effective since the active agent has degenerated or deteriorated. To offset instability problems, many other materials such as chelating agents, antioxidants and masking agents are usually included in the formulation.

Typical emulsions are time consuming to prepare, require heating, are produced in multiple phases, are slow cooling, and often require high shear conditions to get the particle size small enough for maximum stability. Larger batches may require 8 to 24 hours to process and can take several days to set up. It is also often difficult to control the process parameters for preparing the emulsion. If any factors such as the heating, cooling or mixing rates are not carefully duplicated, the preparation may have different properties than the preceding batches of the same product. As a result, the stability of the emulsion may vary from batch to batch. Often the difference of a single parameter is significant enough to cause the product to be outside the established optimum specifications. These batches then have to be either discarded or re-worked.

The lack of reproducibility is especially problematic when the product contains a physiologically active agent. Lack of reproducibility can effect product performance and end user satisfaction. The lack of reproducibility also results in products having different aesthetic properties which the end user will perceive as a lack of quality and will ultimately lead to consumer dissatisfaction or reduced compliance.

Emulsions are typically expensive to manufacture. This is due to a variety of factors including the energy to heat the batch, the specialized equipment required to process the emulsion, such as specialized pumps and cooling/heating equipment, and the time the process ties up equipment and personnel. Moreover, such emulsions cannot be easily processed or customized at the point of purchase. Since most skin care medicaments are prepackaged and have predetermined dosages, dermatologists cannot readily administer to patients varying dosages of these medicaments. As a result, a patient may need to apply two or more different skin care preparations since a single preparation with all of the prescribed medicaments may not be available.

Some dermatologists prepare their own skin care preparations. These skin care preparations typically have poor aesthetic properties resulting in poor patient compliance. Thus, it would be desirable for dermatologists to be able to quickly and easily prepare skin care preparations having varying dosages of medicaments and an aesthetically pleasing appearance.

Present cosmetic products contain predetermined amounts of active agents. Customers cannot pick and choose which ingredients to include in these products. Many customers do not purchase certain cosmetic products because of an allergic reaction with one or more of the ingredients included in the product. For example, many customers are allergic to various fragrances. It would therefore be advantageous to prepare the cosmetic product at the point of sale without the fragrances. Also, customers may have to apply two or more different cosmetic products to get a desired effect since a single product with the desired combination of active agents and/or aesthetic modifying agents may not be on the market. Many cosmetic products are sold in only one form, such as a spray, gel or lotion. Customers, however, may prefer other forms of the cosmetic product.

Prior to the present invention, it was not practical to prepare custom cosmetic products at the point of sale. The preparation of most current cosmetic products require heating, other energy expensive processes, and/or large industrial equipment. As a result, it was not economically feasible to prepare custom cosmetic products at the point of sale. Furthermore, active ingredients which are heat sensitive and oil soluble could not readily be incorporated into cosmetic products by conventional heating without partially or completely degrading the active ingredient.

For the foregoing reasons, there is a need for a substantially surfactant free cosmetic product which can be prepared at the point of sale. Also, there is a need for a method of preparing such a cosmetic product which is fast and does not require heating or other expensive processing techniques.

### SUMMARY OF THE INVENTION

The present invention relates to a composition for topical, oral, nasal, anal, ophthalmic, or vaginal application comprising a base composition and at least one dispersion comprising suspended particles of a hydrophobic active agent, a hydrophobic adjuvant, or a combination thereof. The base composition comprises a rheology modifying agent and water. The composition is substantially free of emulsifying surfactants. The suspended particles generally have a diameter less than 500 or 1,000 nm.

Another embodiment is a method of preparing a topical dispersion comprising mixing the aforementioned base composition with the aforementioned dispersion. Mixing may be performed with a propeller mixer or manually, *i.e.,* by hand. Preferably, the base composition is premanufactured. Since the topical dispersion is simple and quick to prepare, custom cosmetic ecompositions may be prepared at the point of sale for customers in minutes. Prior to the present invention, such products would take hours to be prepared.

The present invention further relates to a method of preparing a composition for topical, oral, nasal, anal, ophthalmic, or vaginal application comprising mixing a base composition with at least one dispersion comprising suspended particles of a hydrophobic active agent, a hydrophobic adjuvant, or a combination thereof. The base composition comprises a rheology modifying agent and water. Preferably, the base composition is premanufactured. Thecomposition is substantially free of emulsifying surfactants and the suspended particles have a diameter less than 500 or 1000 nm. Mixing may be performed with a propeller mixer or manually, *i.e.,* by hand using a spatula or other similar device. Since the composition is simple and quick to prepare, custom cosmetic compositions may be prepared at the point of sale for customers in minutes. Prior to the present invention, such products would take hours to be prepared. Furthermore, the method of the present invention is significantly more efficient (*i.e.* less expensive and faster) than conventional methods for preparing emulsion-based compositions.

### BRIEF DESCRIPTION OF THE DRAWINGS

Fig. 1 is a flowchart generally illustrating the method according to one aspect of the invention;
Fig. 2 is a block diagram of a system for implementing the method; and
Fig. 3 is a flowchart generally illustrating the method according to this aspect of the invention.

### DETAILED DESCRIPTION OF THE INVENTION

The present invention relates to a method of preparing a composition for topical, oral, nasal, anal, ophthalmic, or vaginal application comprising mixing a base composition with at least one dispersion comprising suspended particles of a hydrophobic active agent, a hydrophobic adjuvant, or a combination thereof. The base composition comprises a rheology modifying agent and water.

The base composition is premanufactured, *i.e,* prepared at a location remote from where the mixing step is performed or prepared in large quantities. The term "large quantities" is herein defined as a quantity greater than that needed to produce a single final product and is preferably many multiples times that. The base composition is typically premanufactured in large batches. When the base composition is prepared at a location remote from where the mixing step is performed, it may be dehydrated to form a dry powder or gel in order to decrease transportation costs and ease transport and hydrated at the location where the mixing step is performed.

Other active agents and adjuvants, such as those described in *Remington's Pharmaceutical Sciences,* 19^{th} Edition, A. R. Gennaro (1995) and the *International Cosmetic Ingredient Dictionary and HandbooK,* 7^{th} Edition (1997), published by The Cosmetic, Toiletry, and Fragrance Association (both of which are hereby incorporated by reference), may be mixed with the base composition and dispersion.

Generally, essentially all hydrophobic ingredients to be included in the final composition are added as dispersions (*i.e.* a dispersion of the hydrophobic ingredient is prepared before it is mixed with the base composition and the dispersion). Without being bound by any theory, it is believed that the suspended particles in the dispersions made essentially without surfactants have a charge at their surface resulting from the processing conditions needed to make the dispersions. These mini cells (suspended particles) tend to repel one another. Mini cells made with two or more oils will also not interact because of the repulsive force.

Mixing is generally performed at a temperature of from 15 to 30° C, preferably at a temperature of from 20 to 30°C, and most preferably at ambient temperature. Since the hydrophobic active agent or hydrophobic adjuvant is added to the base composition as a dispersion, heating and other expensive processing steps are not required to obtain a homogenous final composition. Preferably, the composition is not heated during preparation. Generally, mixing is performed at ambient pressure.

Emulsifying surfactants are preferably not added to the composition. As a result, the composition is substantially free of emulsifying surfactants. The composition comprises less than 3% by weight and more preferably less than 1% by weight of emulsifying surfactants, based upon 100% weight of total composition.

The composition may be prepared as a cream, gel, lotion, serum or spray.

Since the method of the present invention may be used to rapidly prepare new formulations (*e.g.* within 5-10 minutes), it can be applied to decrease the cycle time for formulating and manufacturing new formulations. Most typical emulsion-based formulations take hours to be prepared. Additionally, manufacturing formulations according to the method of the present invention is significantly less expensive than conventional manufacturing techniques for emulsion-based compositions. The method of the present invention is particularly applicable to combinatorial methods of formulating.

The present invention further relates to a composition for topical, oral, nasal, anal, ophthalmic, or vaginal application comprising a base composition and at least one dispersion comprising suspended particles of a hydrophobic active agent, a hydrophobic adjuvant, or a combination thereof. The base compositioncomprises a rheology modifying agent and water. The composition is substantially free of emulsifying surfactants. The suspended particles generally have a diameter less than 500 or 1,000 nm.

The composition is substantially free of emulsifying surfactants. The composition comprises less than 3% by weight and more preferably less than 1% by weight of emulsifying surfactants, based upon 100% weight of total composition.

The composition of the present invention may be prepared by mixing the base composition with the dispersion containing at least one of a hydrophobic active agent or a hydrophobic adjuvant. Preferably, the base composition is premanufactured, *i.e.*, prepared at a location remote from where the mixing step is performed or prepared in large quantities. The term "large quantities" is herein defined as a quantity greater than that needed to produce a single final product and is preferably many multiples times that. The base composition is typically premanufactured in large batches.

The dispersion is generally a homogenous fluid which is stable for a commercially relevant period of time. The dispersion typically remains stable for at least 2 weeks and preferably at least 2 months.

According to a preferred embodiment, the dispersion is prepared by mixing from 0.1% to 70% by weight of hydrophobic active agent and/or hydrophobic adjuvant with from 30% to 99.9% by weight of aqueous phase under high pressure and high shear conditions, based upon 100% weight of total dispersion. The aqueous phase contains water and, optionally, other hydrophilic adjuvants. More preferably, the mixing is performed with shearing at a pressure of from 9,000 to 25,000 psi to form a dispersion having an average particle size ranging from 50 to 500 nm.

The present invention further relates to a method for treating topical, oral, nasal, anal, ophthalmic or vaginal disorders with the composition of the present invention.

### Rheology Modifying Agents

The base composition comprises a rheology modifying agent and water.

Rheological modifying agents within the scope of the invention include any substance which increases or decreases the viscosity of the sunscreen formulation. Suitable rheology modifying agents include, but are not limited to, phosphorylated starch derivative, carbohydrate based rheology modifying agents, polymeric and copolymeric rheology modifying agents, inorganic rheology modifying agents, protein rheology modifying agents, polypeptide rheology modifying agents, and any combination of any of the foregoing.

The term "phosphorylated starch derivative" includes, but is not limited to, starches containing a phosphate group. Suitable phosphorylated starch derivatives include, but are not limited to, hydroxyalkyl starch phosphates, hydroxyalkyl distarch phosphates, and any combination of any of the foregoing. Non-limiting examples of hydroxyalkyl starch phosphates and hydroxyalkyl distarch phosphates include hydroxyethyl starch phosphate, hydroxypropyl starch phosphate, hydroxypropyl distarch phosphate (including sodium hydroxypropyl starch phosphate), and any combination of any of the foregoing.

Non-limiting examples of suitable carbohydrate based rheology modifying agents include algin and derivatives and salts thereof, such as algin, calcium alginate, propylene glycol alginate, and ammonium alginate; carrageenan *(Chondrus crispus)* and derivatives and salts thereof, such as calcium carrageenan and sodium carrageenan; agar; cellulose and derivatives thereof, such as carboxymethyl hydroxyethylcellulose, cellulose gum, cetyl hydroxyethylcellulose, hydroxyethylcellulose, hydroxypropylcellulose, hydroxypropyl methylcellulose, methylcellulose, ethylcellulose, and cellulose gum; chitosan and derivatives and salts thereof, such as hydroxypropyl chitosan, carboxymethyl chitosan, and chitin; gellan gum; guar (*Cyanopsis tetragonoloba*) and derivatives thereof, such as guar hydroxypropyltrimonium chloride and hydroxypropyl guar; hyaluronic acid and derivatives thereof, such as sodium hyaluronate; dextran and derivatives thereof; dextrin; locust bean *(Ceratonia siliqua)* gum; mannans and derivatives thereof, such as C₁₋₅ alkyl galactomannan; starches, such as starch polyacrylonitrile copolymer-potassium salt and starch polyacrylonitrile copolymer-sodium salt; pectin; sclerotium gum; tragacanth *(Astragalus gummifer)* gum; xantham gum and derivatives thereof; and any combination of any of the foregoing.

Non-limiting examples of suitable polymeric and copolymeric rheology modifying agents include acrylates, methacrylates, polyethylene and derivatives thereof, and any combination of any of the foregoing. Suitable acrylates and methacrylates include, but are not limited to, carbomer and derivatives and salts thereof, acrylate/C₁₀-C₃₀ alkyl acrylate crosspolymer, acrylate/ceteth-20 itaconate copolymer, acrylate/ceteth-20 methacrylate copolymers, acrylate/steareth-20 methacrylate copolymers, acrylate/steareth-20 itaconate copolymers, acrylate/steareth-50 acrylate copolymers, acrylate/VA crosspolymers, acrylate/vinyl isodecanoate crosspolymers, acrylic acid/acrylonitrogen copolymers, ammonium acrylate/acrylonitrogen copolymers, glyceryl polymethacrylate, polyacrylic acid, PVM/MA decadiene crosspolymer, sodium acrylate/vinyl isodecanoate crosspolymers, sodium carbomer, ethylene/acrylic acid copolymer, ethyleneNA copolymer, acrylate/acrylamide copolymer, acrylate copolymers, acrylate/hydroxyester acrylate copolymers, acrylate/octylarylamide copolymers, acrylate/PVP copolymers, AMP/acrylate copolymers, butylester of PVM-MA copolymer, carboxylate vinylacetate terpolymers, diglycol/CHDM/isophthalates/SIP copolymer, ethyl ester of PVM-MA copolymer, isopropyl ester of PVM-MA copolymer, octylacrylamide/acrylate/butylaminoethyl methacrylate copolymers, polymethacrylamidopropyltrimonium chloride, propylene glycol oligosuccinate, polyvinylcaprolactam, PVP, PVP/dimethylaminoethylmethacrylate copolymer, PVP/DMAPA acrylate copolymers, PVP/carbamyl polyglycol ester, PVPNA copolymer, PVP/VA vinyl propionate copolymer, PVP/vinylcaprolactam/DMAPA acrylate copolymers, sodium polyacrylate, VA/butyl maleate/isobornyl acrylate copolymers, VZ/crotonates copolymer, VA/crotonates vinyl neodecanoate copolymer, VA crotonates/vinyl propionate copolymer, vinyl caprolactam/PVP/dimethylaminoethylmethacrylate copolymer, and any combination of any of the foregoing.

Non-limiting examples of suitable inorganic thickening agents include clays and derivatives thereof, silicates, silicas and derivatives thereof, and any combination of any of the foregoing. Suitable clays and derivatives thereof include, but are not limited to, bentonite and derivatives thereof, such as quaternium-18 bentonite; hectorite and derivatives thereof, such as quaternium-18 dectorite; montmorillonite; and any combination of any of the foregoing. Suitable silicates include, but are not limited to, magnesium aluminum silicate, sodium magnesium silicate, lithium magnesium silicate, tromethamine magnesium aluminum silicate, and any combination of any of the foregoing. Suitable silicas and derivatives thereof include, but are not limited to, hydrated silica, hydrophobic silica, and any combination of any of the foregoing.

Suitable protein and polypeptide rheology modifying agents include, but are not limited to, proteins and derivatives and salts thereof, polypeptides and derivatives and salts thereof, and any combination of any of the foregoing. Non-limiting examples of protein and polypeptide rheology modifying agents include albumin, gelatin, keratic and derivatives thereof, fish protein and derivatives thereof, milk protein and derivatives thereof, wheat protein and derivatives thereof, soy protein and derivatives thereof, elastin and derivatives thereof, silk protein and derivatives thereof, and any combination of any of the foregoing.

Preferred rheology modifying agents include, but are not limited to, carbomer, acrylate/alkyl acrylate crosspolymers, acrylate/vinyl isododecanoate crosspolymer, xantham gum, locust bean gum, guar gum, and any combination of any of the foregoing. A more preferred combination of rheology modifying agents comprises carbomer and an acrylate/alkyl acrylate copolymer, such as an acrylate/C₁₀-C₃₀ alkyl acrylate crosspolymer. According to the International Cosmetic Ingredient Dictionary and Handbook (7^{th} Ed., The Cosmetic, Toiletry, and Fragrance Association), carbomer is a homopolymer of acrylic acid crosslinked with an allyl ether of pentaerythritol, an allyl ether of sucrose, or an allyl ether of propylene. The term "acrylate/alkyl acrylate crosspolymer" includes, but is not limited to, copolymers of alkyl acrylates with one or more monomers of acrylic acid, methacrylic acid, or one of their short chain (i.e. C₁₋₄ alcohol) esters, wherein the crosslinking agent is, for example, an allyl ether of sucrose or pentaerytritol. Preferably, the alkyl acrylates are C₁₀-C₃₀ alkyl acrylates. Examples of such copolymers include, but are not limited to, those commercially available as Carbopol^{™} 1342, Carbopol^{™} 1382, Pemulen^{™} TR-1, and Pemulen^{™} TR-2, from Goodrich Specialty Chemicals of Cleveland, OH.

Preferred rheological modifying agents include, but are not limited to hydrophilic gelling agents, such as carboxyvinyl polymers (carbomer), acrylic copolymers (*e.g.* acrylate/alkyl acrylate copolymers), polyacrylamides, polysaccharides (*e.g*. hydroxypropylcellulose), natural gums, clays, and any combination of any of the foregoing.

Preferably, the cosmetic base contains at least two different rheology modifying agents. Preferred combinations of rheology modifying agents include, but are not limited to, hydroxypropyl distarch phosphate and carbomer; guar hydroxypropyltrimonium chloride and hydroxypropyl guar; sodium hydroxypropyl starch phosphate and carbomer; and hydroxypropyl methylcellulose and pectin.

Generally, the final base composition contains from 0.01 to 35% by weight, preferably from 0.4 to 10% by weight, and more preferably from 0.4 to 6% by weight of the rheological modifying agent, based upon 100% weight of total composition. Typically, the rheology modifying agent is combined with water or water plus, a water soluble cosolvent. The base composition may be prepared by methods known in the art.

The base composition preferably contains a preservative.

### Hydrophobic Active Agent or Hydrophobic Adjuvant Dispersion

A hydrophobic active agent or hydrophobic adjuvant of the present invention is an active agent or adjuvant which has a non polar property which makes it essentially insoluble in water or water and polar solvent solution. Hydrophobic active agents and hydrophobic adjuvants of the present invention include, but are not limited to, partially and fully hydrophobic active agents and partially and fully hydrophobic adjuvants. For example, hydrophobic active agents encompassed by the present invention include compounds and complexes which contain a hydrophobic moiety. The dispersion is generally a homogenous fluid which is stable for a commercially relevant period of time. The dispersion typically remains stable for at least 2 weeks and preferably at least 2 months

The composition of the present invention may also include non-hydrophobic active agents and non-hydrophobic adjuvants.

The dispersion containing the suspended particles generally contains from 0.01 to 70% by weight of oil, based upon 100% weight of total dispersion. Preferably, the dispersion contains from 1.0 to 50% by weight of oil, based upon 100% weight of total dispersion. The oil componentofthe composition may include active agents and adjuvants which are oils.

The dispersion is a suspension of liquid or solid particles of colloidal size or larger in a liquid medium. Generally, the dispersion contains suspended particles, such as oil particles (or oil droplets), having a diameter greater than 1000 nm. The diameter of the suspended particles preferably ranges from about 50 nm to 500 nm and more preferably from 250 to 500 nm. Preferably, the oil droplets contain one or more lipophilic materials. The oil droplets may have a charge as determined by zeta potential measurements. The oil droplets may be prepared by microfluidzation or ultra high shear mixing, such as that described in U.S. Patent No. 6,159,442. Preferred oil containing dispersions are sold under the tradename Sansurf^{™} by Collaborative Laboratories, Inc. of East Setauket, NY. And Dermasomes□ by Microfluidics Corp. of Newton, MA.

According to a preferred embodiment, the dispersion is prepared by mixing from 0.1% to 70% by weight of hydrophobic active agent and/or hydrophobic adjuvant with from 30% to 99.9% by weight of aqueous phase under high pressure, high shear or high pressure and high shear conditions, based upon 100% weight of total dispersion. The aqueous phase contains water and, optionally, other hydrophilic adjuvants. More preferably, the mixing is performed with shearing at a pressure of from 9,000 to 25,000 psi to form a dispersion having an average particle size ranging from 50 to 500 nm.

### Active Agents

Suitable active agents include, but are not limited to, anti-acne agents, antimicrobial agents, antiinflammatory agents, analgesics, antietythemal agents, antipruritic agents, antiedemal agents, antipsoriatic agents, antifungal agents, skin protectants, sunscreen agents, vitamins, antioxidants, scavengers, antiirritants, antibacterial agents, antiviral agents, antiaging agents, protoprotection agents, hair growth enhancers, hair growth inhibitors, hair removal agents, antidandruff agents, anti-seborrheic agents, exfoliating agents, wound healing agents, anti-ectoparacitic agents, sebum modulators, immunomodulators, hormones, botanicals, moisturizers, astringents, sensates, antibiotics, anesthetics, steroids, tissue healing substances, tissue regenerating substances, amino acids, ceramides, and any combination of any of the foregoing.

Preferred anti-acne agents include, but are not limited to, salicylic acid, retinoic acid, alkyl alpha hydroxy acid, benzyl peroxide, sodium sulfacetamide, clindamycin, and any combination of any of the foregoing. Preferred combinations of anti-acne agents to be incorporated in the composition include salicylic acid and retinoic acid; sodium sulfacetamide and clindamycin; salicylic acid and clindamycin; salicylic acid, alkyl alpha hydroxy acid, and tetrahydrozoline.

Suitable antimicrobial agents include, but are not limited to, benzalkonium chloride, benzethonium chloride, chlorhexidine gluconate, chloroxylenol, cloflucarban, fluorosalan, hexachlorophene, hexylresorcinol, iodine complex, iodine tincture, para-chloromercuriphenol, phenylmercuric nitrate, thimerosal, vitromersol, zyloxin, triclocarban, triclosan, methyl-benzethonium chloride, nonyl phenoxypoly(ethyleneoxy) ethanol-iodine, para-chloro-meta-xylenol, triclorcarban, undecoylium chloride-iodine complex, and any combination of any of the foregoing.

Suitable antiinflammatory agents include, but are not limited to, alidoxa, allantoin, aloe vera, aluminum hydroxide, bismuth subnitrate, boric acid, calamine, casein, cellulose, microporous, cholecatciferol, cocoa butter, cod liver oil, colloidal oatmeal, dexpanthenol, dimethicone, glycerin, kaolin, lanolin, live yeast cell derivative, mineral oil, peruvian balsam, petrolatum, protein hydrolysate, racemethionine, shark liver oil, sodium bicarbonate, sulfur, talc, tannic acid, topical starch, vitamin A, vitamin E, white petrolatum, zinc acetate, zinc carbonate, zinc oxide, hydrocortisone, betamethasone, ibuprofen, indomethicin, acetyl salicylic acid, tacrolimus, flucoinolone acetonide, sodium sulfacetamide, and any combination of any of the foregoing.

Suitable analgesics include, but are not limited to, diphenhydramine, tripelennamine, benzocaine, dibucaine, lidocaine, tetracaine, camphor, menthol, phenol, resorcinol, matacresol, juniper tar, methylsalicylate, turpentine oil, capsicum, methyl nicotinate, b-glucan, and any combination of any of the foregoing.

Suitable antietythermal agents include, but is not limited to, tetrahydrozoline and hydracortisone.

Suitable antipruritic agents include, but are not limited to, benadryl, pramoxine, antihistamines, and any combination of any of the foregoing.

Suitable antiedemal agents, include, but are not limited to, pregnenalone acetate, tanin glyrosides, and any combination of any of the foregoing.

Suitable antipsoriatic agents include, but are not limited to, caleipotriene, coal tar, anthralin, vitamin A, and any combination of any of the foregoing. Preferred combinations of antipsoriatic agents include, but are not limited to, hydrocortisone, retinoic acid, and alkyl alpha hydroxy acid; dovonex, salicylic acid, and a sunscreen agent; indomethicin, salicylic acid, and urea; anthralin and salicylic acid; and anthralin and indomethicin. Other suitable antipsoriatic agents include, but are not limited to, caleipotriene, coal tar, anthralin, vitamin A, and any combination of any of the foregoing.

Suitable antifungal agents include, but are not limited to, clioquinol, haloprogin, miconazole nitrate, clotrimazole, metronidazole, toinaftate, undecylenic acid, iodoquinol, and any combination of any of the foregoing.

Suitable skin protectants include, but are not limited to, cocoa butter, dimethicone, petrolatum, white petrolatum, glycerin, shark liver oil, allantoin, and any combination of any of the foregoing.

Suitable sunscreen agents include, but are not limited to, ethylhexyl methoxycinnamate, avobenzone, benzophenone-3, octacrylene, titanium dioxide, zinc oxide, and any combination of any of the foregoing.

Suitable antioxidants include, but are not limited to, scavengers for lipid free radicals and peroxyl radicals, quenching agents, and any combination of any of the foregoing. Suitable antioxidants include, but are not limited to, tocopherol, BHT, beta carotene, vitamin A, ubiquinol, ferulic acid, azelaic acid, thymol, catechin, sinapic acid, lactoferrin, rosmariquinone, hydroxytyrosole, sesamol, 2-thioxanthine, nausin, malvin, carvacone, chalcones, glutathione isopropyl ester, xanthine, melanin, guanisone, lophorphyrins, 8-hydroxyxanthine, 2-thioxanthione, vitamin B₁₂, plant alkaloids, catalase, quercetin, tyrosine, SOD, cysteine, methionine, genistein, NDG, procyanidin, hamamelitannin, ubiquinone, trolox, licorice extract, propyl gallate, sinapic acid, and any combination of any of the foregoing. Suitable vitamins include, but are not limited to, vitamin E, vitamin A palmitate, vitamin D, vitamin F, vitamin B₆, vitamin B₃, vitamin B₁₂, vitamin C, ascorbyl palmitate, vitamin E acetate, biotin, niacin, DL-panthenol, and any combination of any of the foregoing.

A preferred sunscreen agent is a mixture of ethylhexyl methoxycinnamate, butyl methoxydibenzoylmethane, and water, and is available as Solarease™ from Collaborative Laboratories, Inc. of East Setauket, NY.

Suitable amino acids include, but are not limited to, glycine, serine, and any combination of any of the foregoing.

### Aesthetic Modifying Agents

The composition preferably includes at least one aesthetic modifying agent. An aesthetic modifying agent is a material which imparts desirable tactile, olfactory, taste or visual properties to the surface to which the composition is applied. The aesthetic modifying agent may be hydrophobic or hydrophilic. The aesthetic modifying agent is preferably hydrophobic and is more preferably an oil, wax, solid or paste.

A dispersion of one or more hydrophobic aesthetic modifying agents is preferably prepared before the hydrophobic aesthetic modifying agents are incorporated into the composition. The hydrophobic aesthetic modifying agents may be dispersed into an aqueous phase by methods, such as ultra high shear mixing and microfluidization.

The final composition may be prepared by mixing the dispersions containing the hydrophobic aesthetic modifying agents with the base composition and any other adjuvants. Since the hydrophobic aesthetic modifying agents are added to the base composition as dispersions, heating and other expensive processing steps are not required to obtain a homogenous final composition.

An example of an aesthetic modifying agent is a mono, di, tri or poly alkyl ester or ether of a di, tri, or polyhydroxy compound, such as ethylene glycol, propylene glycol, glycerin, sorbitol or other polyol compound. Examples of such esters and ethers include, but are not limited to, saturated and unsaturated, linear and branched vegetable oils, such as soybean oil, babassu oil, castor oil, cottonseed oil, chinese tallow oil, crambe oil, perilla oil, danish rapeseed oil, rice bran oil, palm oil, palm kernel oil, olive oil, linseed oil, coconut oil, sunflower oil, safflower oil, peanut oil and corn oil. Preferred saturated and unsaturated vegetable oils are those having fatty acid components with 6 to 24 carbon atoms. A more preferred vegetable oil is soybean oil.

An example of a hydrophobic aesthetic modifying agent is a compound having the formula CₙH₍₂ₙ₊₂₋ₘ₎ where n is an integer greater than or equal to 6 and m is 0 or an even integer no greater than n. Such compounds include, but are not limited to, saturated and unsaturated, linear, branched, and cyclic hydrocarbon chains. Preferred examples of such compounds include, but are not limited, mineral oil, petrolatum, permethyl fluids, polybutylenes, and polyisobutylenes.

Another example of a hydrophobic aesthetic modifying agent has the formula 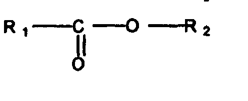 or the formula 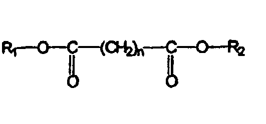 where R₁ is a saturated or unsaturated, linear, branched or cyclic C₁-C₂₄ alkyl; R₂ is hydrogen or a saturated or unsaturated, liner, branched or cyclic C₁-C₂₄ alkyl; and n is an integer from 0 to 20. Examples of such aesthetic modifying agents include, but are not limited to, isopropyl palmitate and diisopropyl adipate.

Yet another aesthetic modifying agent is silicone. Silicone may provide lubrication and/or shine to the composition. Preferably, the silicone is insoluble in water. Suitable water-insoluble silicone materials include, but are not limited to, polyalkylsiloxanes, polyarylsiloxanes, polyalkylarylsiloxanes, polysiloxane gums and polyethersiloxane copolymers. Examples of suitable silicone materials are disclosed in U.S. Patent Nos. 4,788,006; 4,341,799; 4,152,416; 3,964,500; 3,208,911; 4,364,837 and 4,465,619.

Another suitable hydrophobic material which can be suspended in the composition has the formula 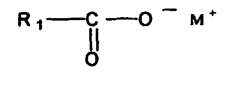 where R₁ is a saturated or unsaturated, linear, branched or cyclic alkyl having 2 to 24 carbon atoms; M⁽⁺⁾ is N⁺R₂R₃R₄R₅; R₂, R₃ and R₄ are hydrogen or a saturated or unsaturated, linear or branched alkyl or hydroxyalkyl having from 1 to 10 carbon atoms; and R₄ is a saturated or unsaturated, linear, branched or cyclic alkyl or substituted alkyl having 2 to 24 carbon atoms. An example of such a material is lauramine oleate.

Another aesthetic modifying agent is a polymer formed by polymerization of alkylene oxide monomers of the formula 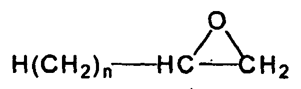 where n is an integer from 0 to about 24. The polymer may be either a homogenous polymer or a copolymer. Examples of such homogenous polymers include, but are not limited to, polypropylene oxide and polybutylene oxide. Generally, the molecular weight of these polymers ranges from about 100 to about 10,000 daltons. Additionally, these polymers may be reacted with mono or polyhydroxyalkyl alcohol, such as UCON fluids available from the Union Carbide Chemical Company, or with a saturated or unsaturated, linear, branched or cyclic C₁-C₂₄ alkyl.

### Other Adjuvants

Other suitable adjuvants include but are not limited to pH adjusters, emollients, conditioning agents, chelating agents, gelling agents, viscosifiers, colorants, fragrances, odor masking agents, UV stabilizer, preservatives, and any combination of any of the foregoing. Preferred pH adjusters include, but are not limited to, aminomethyl propanol, aminomethylpropane diol, triethanolamine, citric acid, sodium hydroxide, acetic acid, potassium hydroxide, lactic acid, and any combination of any of the foregoing.

Suitable conditioning agents include, but are not limited to, cyclomethicone, petrolatum, dimethicone, dimethiconol, silicone, quaternary amines and any combination of any of the foregoing.

The composition preferably contains less than 0.5% by weight of preservatives, based upon 100% weight of total composition. More preferably, the composition contains from 0.25 to 0.5% by weight of preservatives, based upon 100% weight of total composition.

### Systems and Methods for Production of Customized Compositions

As will be appreciated by those of skill in the art, because of the long time required to manufacture an adequate base composition for use in pharmaceutical and cosmetic compositions, it has not been considered possible to produce such compositions on demand or in wide variety at a rapid pace. In accordance with a further aspect of the invention, a method and system for producing such a customized composition for using the above described formulations (specific examples of which are given below) or possibly other similar formulations is provided. The method is suitable for manual use, i.e., in a doctor's office, and is also well suited for use in an on-demand customized manufacturing system.

According to a primary element of this aspect of the invention, a base composition comprising a rheology modifying agent and water is provided for use in a pre-mixed format at the manufacturing location. This eliminates the need to provide specialized mixing equipment at that location and to wait the long period of time (typically several hours) conventionally needed to prepare the base composition. In one embodiment, the base composition can be pre-mixed at the manufacturing location, e.g., in bulk, in advance of an expected manufacturing run. Alternatively, the base composition can be prepared at a location remote from the manufacturing location and delivered in advance. Also provided at the manufacturing location are a plurality of dispersions, each comprising suspended particles of one or more hydrophobic active agents, hydrophobic adjuvants, or combination thereof. Each dispersion has a predefined characteristic or property, such as a medicinal effect. One or more aesthetic modifying agents may also be provided.

At least one of the available dispersions is selected in accordance with the desired characteristics, e.g., as indicated by a customer order, of the composition to be manufactured. The appropriate quantities of the selected dispersion(s) and the base composition are then mixed at a temperature of from 20 to 30 degrees Celsius to produce the final product. More preferably, they are mixed at an ambient or room temperature. One or more aesthetic modifying agents may also be selected, by default or in accordance with the order, and added to the mix in appropriate quantities. Advantageously, because the base composition is pro-mixed, and the additive is provided as a hydrophobic dispersion, this mixing step can be accomplished very rapidly, typically in five minutes or less, making the process suitable for use in manufacturing a large variety of compounds in a short period of time or for manufacturing individual orders on-demand for near-instant delivery.

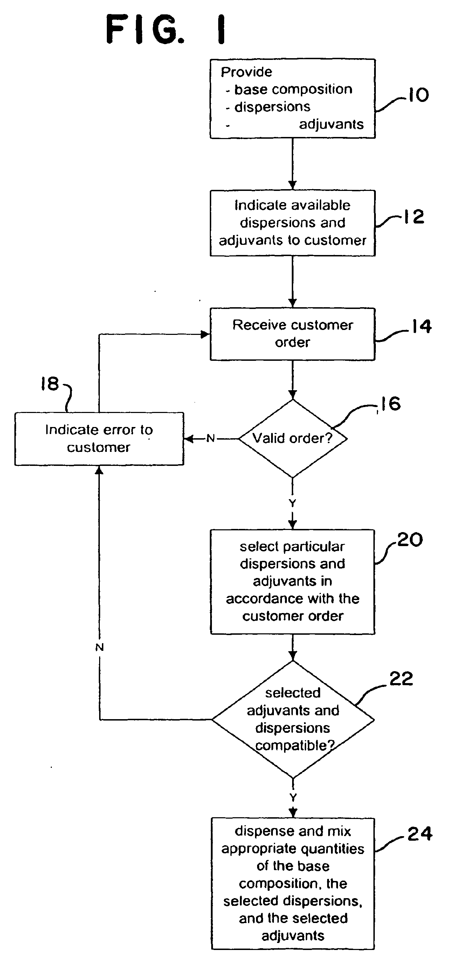

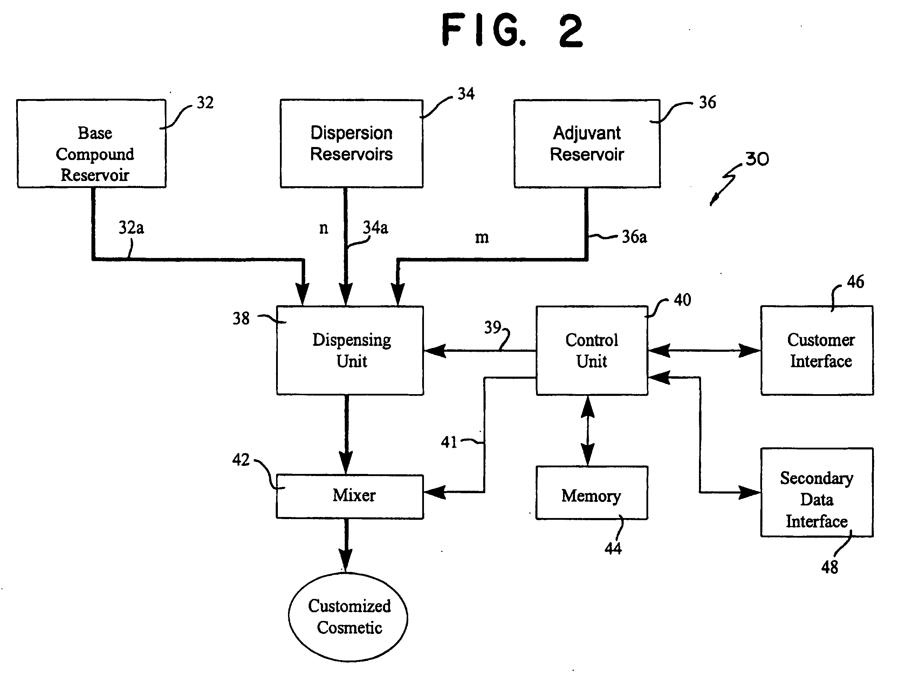

A particular method for producing a customized composition for at least one of topical, oral, nasal, anal, ophthalmic, and vaginal application, which method is suitable for use in either a distributed Internet-based system or an on-demand kiosk manufacturing system will now be discussed. Fig. 1 is a flowchart generally illustrating the method according to one aspect of the invention. Fig. 2 is a block diagram of a system for implementing the method. The system may be a remotely located manufacturing facility 30 which receives customer orders, e.g., through the Internet, or may be housed within a suitable kiosk to provide on-demand manufacturing and delivery. Such a kiosk may be located in a point-of-sale establishment, such as a department store. Alternatively, the kiosk or manufacturing facility may be integrated into a pharmacy. This configuration permits customer orders to be entered by a doctor on behalf of the customer, e.g., through an appropriate Internet web-site. The ordered composition will then automatically be produced at the pharmacy for subsequent pick-up by the customer.

Turning to Figs. 1 and 2, at a first location, initially (a) a base composition comprising a rheology modifying agent and water; (b) a plurality of dispersions; and (c) adjuvants are provided at a first location, generally the manufacturing site. (Step 10). The adjuvants may include one or more aesthetic modifying agents may also be provided. The base composition, dispersions, and adjuvants are provided in reservoirs 32, 34, 36 housed within the manufacturing facility 30 and connected via appropriate conduits 32a, 34a, 36a to a dispensing unit 38. Dispensing unit 38 is a configured to dispense measured amounts of selected ones of the provided components in response to input control signals 39 produced by a control unit 40, such as a computer-based system with appropriate operating programs stored in memory 44. Appropriate dispensing units and control units will be known to those of skill in the art.

A customer order is received at the manufacturing facility 30 (step 14). The order is received through an appropriate customer interface 46. When system 30 is a manufacturing facility remote from the customer, interface 46 is preferably a two-way Internet connection. When system 30 is a kiosk based-facility, the customer interface 46 will typically comprise a video display unit and an entry system, such as a keyboard or touch-screen. Preferably, prior to receiving the customer order, the customer is provided with an indication as to which dispersions and agents are available. (Step 12).

The customer order specifies desired properties of the composition to be manufactured. The properties may be general attributes which are associated to specific dispersions and/or adjuvants by the control unit 40 in accordance with preprogramed database. Alternatively, the properties may correspond directly to the available dispersions and adjuvants. The implementation depends on the expected sophistication of the customer. Once an order has been received, particular dispersions and adjuvants are selected in accordance with the properties specified in the order (step 20). Preferably, the order passes through a verification process, which may be before and/or after the selection step. During verification, the order is checked to make sure that appropriate materials are available to produce the ordered composition(step 16) and that when the selected dispersions and adjuvants are compatible with each other (step 22). If an error is detected, it is communicated to the customer (step 18) and the customer is asked to enter a corrected order. Alternative formulations may also be suggested to the customer prior to receiving an updated order.

Finally, appropriate quantities of the base composition and the selected dispersions and adjuvants are determined, e.g., in accordance with data-tables stored in the memory 44. The control unit 40 then generates appropriate control signals to instruct the dispensing unit 38 to dispense the determined quantities of compounds from the reservoirs 32, 34, 36 and pass them into a mixer 42. The mixer is activated by the control unit 40 for a short period of time to thereby produce the customized product (step 24) which is then packaged and delivered to the consumer, e.g., through the mail or by dispensing it from the kiosk. Because the mixing step 24 is a relatively short procedure, mixing may be performed directly in the container used to dispense the compound.

According to yet a further aspect of the invention, particularly well suited for the kiosk-based system, but also applicable in an Internet-based environment, various order combinations of dispersion and/or adjuvants (i.e., order properties) may be suggested to the customer. The suggestions may be in accordance with customer profile information, such as skin-type, hair and eye color, age, gender, as well as various other physiological parameters and attributes. Suggestions may also make use of information gathered from prior orders or other sources. The suggestions may be an aid to help the customer directly indicate the dispersions, water soluble active agents, and/or adjuvants which are to be added. Alternatively, the suggestions may indicate general properties, without regard to the specific components to be added, which components are identified by the control unit 40 during order processing.

The customer profile information can be entered directly by the customer (or a customer service agent) into a kiosk machine or Internet web interface. Alternatively, profile information can be entered onto an order form which is subsequently scanned into or otherwise entered into the system. In yet a further embodiment, an electronic image of the customer, possibly along with other biometric and physiological measurements, is entered into the system and processed to generate a basic user profile. In addition, suggestions may also be made in accordance with environmental factors, such as the time of year, local weather, and the geographic region the customer is in. For example, if a kiosk is located in the North East during winter, the system may suggest that the customer add a moisturizer. If the kiosk is located in Florida during June, the system may suggest the addition of a sunscreen.

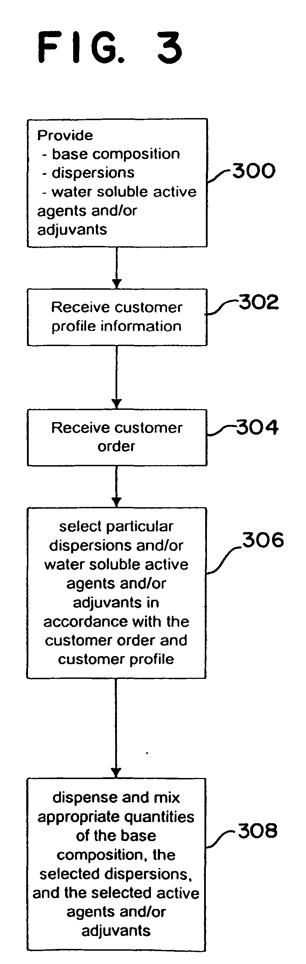

In one embodiment of the invention, the user is shielded from selecting the particular dispersions, etc., which are added to the custom compound. Instead, the user profile information and general property selections made by the user are used to determine the overall composition of the compound. In this embodiment, the processing flow is generally simplified since the control unit 40 can ensure that the dispersions, water soluble active agents, and/or adjuvants which are selected to produce the desired composition properties are available and are compatible with each other. Fig. 3 is a flowchart generally illustrating the method according to this aspect of the invention. As shown, the various source materials are first provided. (Step 300). Next, the customer profile information is received, e.g., from the customer, from a customer database, through a customer profile generation subroutine receiving input from a digital camera, etc. (Step 302). The customer order is the received, perhaps after various compound properties are suggested to the customer in accordance with an analysis of the user profile. (Step 304). In accordance with the composition properties indicated in the customer order and the customer profile, appropriate dispersions, water soluble active agents and/or adjuvants are selected and appropriate quantities to use are determined. (Step 306). Finally, the appropriate quantities of the selected materials are dispensed and mixed. (Step 308). The final product is then packaged and delivered to the customer.

Customer profile data, prior ordering history, and other information may be stored in an appropriate database and retrieved, e.g., based on a customer name or ID, for use in processing subsequent orders. If system 30 is a kiosk-based system, preferably a secondary data interface 48 is provided to permit customer profile information and additional data to be accessed by the kisok, for the kiosk status to be monitored from a remote site, and for the kiosk to send information messages to an appropriate party, e.g., indicating that certain of the reservoirs need refilling, etc. It should be appreciated that while the kiosk systems are stand-alone units, some or all of the data processing and decision making requirements may be off-loaded from the kiosk to a centralized server accessible through the secondary data interface 48.

The following examples are intended to describe the present invention without limitation.

### Examples 1-3

These examples demonstrate the flexibility of the system to produce multiple product forms from the same base ingredients if needed. Serum, lotion, and cream base compositions having the formulations in Table 1 below were prepared as follows. Deionized water (A) and Germazide MPB were mixed. Structure Zea was sprinkled into the solution. Carbopol 940 was added and the solution was mixed. Triethanolamine and deionized water (B) were added and the solution was mixed to form the cosmetic base composition.

**Table 1**

| Ingredient | Percentage Weight (based upon 100% total weight of cosmetic base) | | |
|---|---|---|---|
| | Example 1 Serum Base | Example 2 Lotion Base | Example 3 Cream Base |
| Structure Zea¹ | 0.75 | 1.50 | 3.00 |
| Germazide^{™} MPB² | 1.50 | 1.50 | 1.50 |
| Carbopol 940³ 2% aqueous solution | 7.50 | 15.00 | 30.00 |
| Triethanolamine (99%) | 0.21 | 0.43 | 0.86 |
| Deionized Water (A) | 85.00 | 78.00 | 63.90 |
| Deionized Water (B) | QS | QS | QS |

| | | | |
|---|---|---|---|
| ¹ - Structure Zea is a hydroxypropyl distarch phosphate and is available as from National Starch and Chemical Co. of Bridgewater, NJ. | | | |
| ² - Germazide^{™} MPB is a mixture of phenoxyethanol, chlorphenesin, glycerin, methylparaben, and benzoic acid and is available from Collaborative Laboratories, Inc. of East Setauket, NY. | | | |

³ - Carbopol 940 is available from Goodrich Specialty Chemicals of Cleveland, OH. The pH of the serum, lotion, and cream bases were 6.67, 6.17, and 6.2, respectively.

### Example 4

This example demonstrates how an oil soluble active can be incorporated into the base giving a surfactant free product. A moisturizing lotion for dry skin with sunscreens having the formulation of Table 2 below was prepared by mixing the ingredients with either a paddle blade or propeller mixer or with hand mixing with a spatula or other similar device.

**Table 2**

| Ingredient | Percentage Weight (based upon 100% total weight of composition) |
|---|---|
| Cream Base of Example 3 | 75.00 |
| Solarease^{™4} | 25.00 |

| | |
|---|---|
| ⁴ - Solarease^{™} is a mixture of ethylhexyl methoxycinnamate, butyl methoxydibenzoylmethane, cyclomethicone, phospholipids, and water and is available from Collaborative Laboratories, Inc. of East Setauket, NY. | |

### Example 5

This example demonstrates how the product in Example 4 can be supplemented with a water soluble aesthetic modifying agent. A moisturizing gel lotion for normal skin having the formulation of Table 3 below was prepared by mixing the ingredients either with a propeller or paddle blade mixer or with hand mixing with a spatula or other similar device.

**Table 3**

| Ingredient | Percentage Weight (based upon 100% total weight of composition) |
|---|---|
| Lotion Base of Example 2 | 60.00 |
| Solarease^{™} | 15.00 |
| Seamollient^{™5} | 25.00 |

| | |
|---|---|
| ⁵ - Seamollient^{™} is a mixture of water, algae extract, chlorphenesin, propylene glycol, sodium dehydroacetate, and phenoxyethanol and is available from Collaborative Laboratories, Inc. of East Setauket, NY. | |

### Example 6

This example shows an example of a formulation containing an oil dispersion of an organic sunscreen. An oil-free moisturizer for oily skin having the formulation of Table 4 below was prepared by mixing the ingredients either with a propeller or paddle blade mixer or with hand mixing with a spatula or other similar device.

**Table 4**

| Ingredient | Percentage Weight (based upon 100% total weight of composition) |
|---|---|
| Lotion Base of Example 2 | 80.00 |
| Solarease^{™} | 15.00 |
| Deionized Water | 5.00 |

### Example 7

This is an example of a cream moisturizer containing a variety of different oil dispersions of aesthetic modifying agents. A cream moisturizer having the formulation of Table 5 below was prepared by mixing the ingredients with either a propeller or paddle blade mixer or with hand mixing with a spatula or other similar device.

**Table 5**

| Ingredient | Percentage Weight (based upon 100% total weight of composition) |
|---|---|
| Cream Base of Example 3 | 60.00 |
| AM500⁶ | 10.00 |
| AM600⁷ | 10.00 |
| AM200⁸ | 15.00 |
| Deionized Water | 5.00 |

| | |
|---|---|
| ⁶- AM500 is a mixture of water, petrolatum, and cyclomethicone and is available from Collaborative Laboratories, Inc. of East Setauket, NY. | |
| ⁷ - AM600 is a mixture of water, cyclopentasiloxane, cyclomethicone phospholipids, and dimethicone/vinyl dimethicone polymer and is available from Collaborative Laboratories, Inc. of East Setauket, NY. | |
| ⁸ - AM200 is a mixture of water, cyclopestasiloxane and phospholipids and is available from Collaborative Laboratories, Inc. of East Setauket, NY. | |

### Example 8

This example shows the compatibility of the system with liposome delivery systems. A dry skin moisturizer having the formulation of Table 6 below was prepared by mixing the ingredients either with a propeller or paddle blade mixer or with hand mixing with a spatula or other similar device.

**Table 6**

| Ingredient | Percentage Weight (based upon 100% total weight of composition) |
|---|---|
| Cream Base of Example 3 | 46.95 |
| Frescolat Type ML⁹ | 0.05 |
| Solarease II¹⁰ | 15.00 |
| AM600 | 8.00 |
| SanSurf Calendula¹¹ | 20.00 |
| AM500 | 8.00 |
| Vitamin A & E liposomes¹² | 0.50 |
| Germazide MPB | 1.50 |

| | |
|---|---|
| ⁹ - Frescolat Type ML is menthyl lactate and is available from Haaram & Reimer Corp. of Springfield, NJ. | |
| ¹⁰ - Solarease II is is a mixture of ethylhexyl methoxycinnamate, butyl methoxydibenzoylmethane, cyclomethicone, phospholipids, and water and is available from Collaborative Laboratories, Inc. of East Setauket, NY. | |
| ¹¹ - Sansurf Calendula is a mixture of cyclopestasiloxane, a propylene glycol based extract of calendula, and is available from Collaborative Laboratories, Inc. of East Setauket, NY. | |
| ¹² - Vitamin A & E liposomes is a mixture of water, phospholipids, tocopheryl acetate, and retinyl palmitate and is available from Collaborative Laboratories, Inc. of East Setauket, NY. | |

### Examples 9 and 10

These examples show the compatibility of creams and lotions containing water soluble active polymers with dispersions of aesthetic modifying agents. Cream and lotion moisturizers having the formulations of Table 7 below were prepared by mixing the ingredients either with a propeller or paddle blade mixer or with hand mixing with a spatula or other similar device.

**Table 7**

| Ingredient | Percentage Weight (based upon 100% total weight of composition) | |
|---|---|---|
| | Example 9 | Example 10 |
| Cream Base of Example 3 | 68.82 | - |
| Lotion Base of Example 2 | - | 80.00 |
| Sansurf Petrolatum-25¹³ | 5.90 | 4.00 |
| Satin Finish¹⁴ | 0.99 | 0.60 |
| AM600¹⁵ | 6.88 | 4.50 |
| Advanced Moisture Complex¹⁶ | 1.97 | 1.00 |
| Halosol (1%) | 1.97 | 1.00 |
| Sansurf Vegepure¹⁷ | 4.42 | 2.80 |
| Sansurf PFMP¹⁸ | 5.61 | 3.80 |
| Sansurf SI¹⁹ | 3.44 | 2.30 |

| | | |
|---|---|---|
| ¹³ - Sansurf Petrolatum-25 is a mixture of water, petrolatum, and cyclomethicone and is available from Collaborative Laboratories, Inc. of East Setauket, NY. | | |
| ¹⁴ - Satin Finish is a mixture of water, phenyl trimethicone, cyclomethicone, dimethiconol, phospholipids, carbomer, and triethanolamine and is available from Collaborative Laboratories, Inc. of East Setauket, NY. | | |
| ¹⁵- AM600 is a mixture of water, cyclopestasiloxane, cyclomethicone, phospholipids, and dimethicone/vinyl dimethicone cross polymer and is available from Collaborative Laboratories, Inc. of East Setauket, NY. | | |
| ¹⁷ - SansurfVegepure is a mixture of water, vegepure, cyclomethicone, dimethiconol, and phospholipids and is available from Collaborative Laboratories, Inc. of East Setauket, NY. | | |
| ¹⁸ - Sansurf PFMP is a mixture of water, perfluoropolymethylisopropylether, and phospholipids and is available from Collaborative Laboratories, Inc. of East Setauket, NY. | | |
| ¹⁹ - Sansurf SI is a mixture of water, phenyl trimethicone, and phospholipids and is available from Collaborative Laboratories, Inc. of East Setauket, NY. | | |

### Example 11

A dry skin sunscreen moisturizer having the formulation of Table 8 below was prepared by mixing the ingredients either with a propeller or paddle blade mixer or with hand mixing with a spatula or other similar device.

**Table 8**

| Ingredient | Percentage Weight (based upon 100% total weight of composition) |
|---|---|
| Cream Base of Example 3 | 70.00 |
| Solarease^{™} | 25.00 |
| Mustard b-Glucan R-25 (Natunola)²⁰ | 2.50 |
| Deionized Water | 2.50 |

| | |
|---|---|
| ²⁰ - Mustard b-Glucan R-25 is water, white mustard (brassica alba) extract and is available from Natunola ofNepean, Ontario, Canada. | |

### Example 12

A dry skin sunscreen moisturizer having the formulation of Table 9 below was prepared by mixing the ingredients with a propeller mixer.

**Table 9**

| Ingredient | Percentage Weight (based upon 100% total weight of composition) |
|---|---|
| Cream Base of Example 3 | 70.20 |
| Frescolat Type ML | 0.05 |
| Solarease^{™} | 25.00 |
| Mustard b-Glucan | 2.50 |
| Dow Corning 1403 Fluid²¹ | 0.75 |
| Germazide MPB | 1.50 |

| | |
|---|---|
| ²¹ - Dow Coming 1403 Fluid is a mixture of dimethicone and dimethiconol and is available from Dow Coming Corp. of Midland, MI. | |

### Examples 13-16

This example shows the ability to produce various lotions without oil that can incorporate extracts of oil absorbing materials. Oil-free moisturizers having the formulations in Table 10 below were prepared by mixing the ingredients either with a propeller or paddle blade mixer or with hand mixing with a spatula or other similar device.

**Table 10**

| Ingredient | Percentage Weight (based upon 100% total weight of composition) | | | |
|---|---|---|---|---|
| | Example 13 | Example 14 | Example 15 | Example 16 |
| Lotion Base of Example 2 | 85.00 | 85.00 | 83.30 | 73.30 |
| Solarease™ | 10.00 | 10.00 | 10.00 | 10.00 |
| Satin Finish | - | - | - | 10.00 |
| Peppermint Extract | 0.20 | - | - | - |
| Frescolat Type ML | - | 0.20 | 0.20 | 0.20 |
| Germazide MPB | - | - | 1.50 | 1.50 |
| Celluflow TA-25²² | - | - | 5.00 | 5.00 |
| Deionized Water | 4.80 | 4.80 | - | - |

| | | | | |
|---|---|---|---|---|
| ²² - Celluflow TA-25 is cellulose acetate and is available from Collaborative Laboratories, Inc., East Setauket, New York. | | | | |

### Examples 17 and 18

Dry skin moisturizing creams and lotions having the formulations in Table 11 below were prepared by mixing the ingredients either with a propeller or paddle blade mixer or with hand mixing with a spatula or other similar device.

**Table 11**

| Ingredient | Percentage Weight (based upon 100% total weight of composition) | |
|---|---|---|
| | Example 17 | Example 18 |
| Lotion Base of Example 2 | - | 63.20 |
| Cream Base of Example 3 | 63.20 | - |
| Frescolat Type ML | 0.05 | 0.05 |
| Solarease^{™} | 25.00 | 25.00 |
| Mustard b-Glucan (Natunola) | 2.50 | 2.50 |
| Dow Coming 1403 Fluid | 0.75 | 0.75 |
| Sansurf GE Silicone 839 SFE | 7.00 | 7.00 |
| Germazide MPB | 1.50 | 1.50 |

### Examples 19-21

Lotions having the formulations in Table 12 below were prepared by mixing the ingredients either with a propeller or paddle blade mixer or with hand mixing with a spatula or other similar device.

**Table 12**

| Ingredient | Percentage Weight (based upon 100% total weight of composition) | | |
|---|---|---|---|
| | Example 19 | Example 20 | Example 21 |
| Lotion Base of Example 2 | 80.00 | 80.00 | 80.00 |
| Sansurf Crodamol W²³ | 20.00 | - | 10.00 |
| Sansurf Lanolin ²⁴ | - | 20.00 | 10.00 |

| | | | |
|---|---|---|---|
| ²³ - Sansurf Crodamol W is a mixture of water, stearyl heptanoate, cyclomethicone, and phospholipids and is available from Collaborative Laboratories, Inc. of East Setauket, NY. | | | |
| ²⁴ - Sansurf Lanolin is a mixture of water, lanolin, cyclomethicone, PEG-4, and phospholipids and is available from Collaborative Laboratories, Inc. of East Setauket, NY. | | | |

### Example 22

A petrolatum spray having the formulation in Table 13 below was prepared by mixing the ingredients with either a propeller or paddle blade mixer or with hand mixing with a spatula or other similar device. This product can be used to provide a barrier film on the skin.

**Table 13**

| Ingredient | Percentage Weight (based upon 100% total weight of composition) |
|---|---|
| Lotion Base of Example 2 | 16.00 |
| Sansurf Petrolatum-50 | 25.00 |
| Germazide MPB | 1.50 |
| Deionized Water | 57.50 |

### Examples 23 and 24

Intensive moisturizing creams having the formulations in Table 14 below were prepared by mixing the ingredients either with a propeller or paddle blade mixer or with hand mixing with a spatula or other similar device.

**Table 14**

| Ingredient | Percentage Weight (based upon 100% total weight of composition) | |
|---|---|---|
| | Example 23 | Example 24 |
| Cream Base of Example 3 | 70.00 | 70.00 |
| Advanced Moisture Complex* | 1.00 | 5.00 |
| Deionized Water | 29.00 | 25.00 |

| | | |
|---|---|---|
| * - The advanced moisture complex is a mixture of glycerine, water, sodium PCA, urea, trichalese, polyquatenium 51 and sodium hyaluronate. | | |

### Examples 25-28

Skin protecting moisturizing creams having the formulations in Table 15 below were prepared by mixing the ingredients either with a propeller or paddle blade mixer or with hand mixing with a spatula or other similar device.

**Table 15**

| Ingredient | Percentage Weight (based upon 100% total weight of composition) | | | |
|---|---|---|---|---|
| | Example 25 | Example 26 | Example 27 | Example 28 |
| Cream Base of Example 3 | 70.00 | 70.00 | 70.00 | 70.00 |
| Dermaguard²⁵ | 10.00 | - | - | 10.00 |
| Advanced Moisture Complex* | - | 10.00 | - | 10.00 |
| Sansurf Petrolatum-25 | - | - | 10.00 | - |
| Deionized Water | 20.00 | 20.00 | 20.00 | 10.00 |

| | | | | |
|---|---|---|---|---|
| *- The advanced moisture complex is a mixture of glycerine, water, sodium PCA, urea, trichalese, Polyquaternium-51 and sodium hyaluronate. | | | | |
| ²⁵ - Dermaguard^{™} is a mixture of water, petrolatum, dimethicone, perfluoropolymethylisopropylether, stearamidopropyl dimethylamine, stearic acid, and tocopherol acetate, and is available from Collaborative Laboratories, Inc. of East Setauket, NY. | | | | |

### Examples 29 and 30

These examples illustrate that the base compositions of the present invention are compatible with low pH compositions. Lotion and cream bases having the formulations in Table 16 below were prepared by mixing the ingredients with a propeller mixer.

**Table 16**

| Ingredient | Percentage Weight (based upon 100% total weight of cosmetic base) | |
|---|---|---|
| | Example 29 Lotion Base | Example 30 Cream Base |
| Carrageenan | 0.75 | 1.50 |
| Sclerotium Gum | 0.75 | 1.50 |
| Glycerin | 1.0 | 2.00 |
| Germazide MPB | 1.5 | 1.50 |
| Deionized Water | 95.0 | 87.5 |
| Propylene Glycol | - | 2.00 |

The pH of the lotion base and cream base were about 4.16 and 3.85, respectively. The specific gravity of the lotion base and cream base were about 1.033 and 1.012 g/cc, respectively.

### Example 31

This example illustrates a cationic base which is compatible with cationic active agents and cationic aesthetic modifying agents. A cosmetic base having the formulation in Table 17 below was prepared as follows. Jaguar HP60 was sprinkled into the deionized water mix until homogenous and smooth. Citric acid was added to thicken the solution and adjust the pH to about 5.22. The specific gravity of the cosmetic base was about 1.015 g/cc.

**Table 17**

| Ingredient | Percentage Weight (based upon 100% total weight of cosmetic base) |
|---|---|
| Jaguar HP60²⁷ | 2.00 |
| Deionized Water | 98.00 |

| | |
|---|---|
| ²⁷ - Jaguar HP60^{™} is hydroxypropyl guar and is available from Rhône-Poulenc of Cranbury, NJ. | |

### Example 32

A cream base having the formulation in Table 18 below was prepared as follows. Deionized water (A), Pure Gel B980, and Germazide MPB were mixed together. Carbopol 940 was added to the soluion and mixed. Triethanolamine and deionized water (B) were added to the solution and mixed.

**Table 18**

| Ingredient | Percentage Weight (based upon 100% total weight of cream base) |
|---|---|
| Pure Gel B980²⁸ | 4.00 |
| Germazide MPB | 1.50 |
| Carbopol 940 (2% Aqueous) | 30.00 |
| Triethanolamine | 0.43 |
| Deionized Water (A) | 56.00 |
| Deionized Water (B) | 8.07 |

| | |
|---|---|
| ²⁸ - Pure Gel B980 is sodium hydroxypropyl starch phosphate and is available from Grain Processing Corporation. | |

### Example 33

This is an example of a cationic gel base. A cosmetic base having the formulation in Table 19 below was prepared as follows. Jaguar C 14S and Jaguar HP8COS were mixed together. While the deionized water was stirred, the mixture of Jaguar C14S and HP8COS was sprinkled into the outer edge of the vortex formed by the rotating water. The solution was mixed.

**Table 19**

| Ingredient | Percentage Weight (based upon 100% total weight of cream base) |
|---|---|
| Jaguar C14S^{™29} | 0.75 |
| Jaguar HP8C0S^{™30} | 0.375 |
| Deionized Water | 98.875 |

| | |
|---|---|
| ²⁹ - Jaguar C14S^{™} is guar hydroxypropyltrimonium chloride and is available from Rhône-Poulenc of Cranbury, NJ. | |
| ³⁰ - Jaguar JP8COS^{™} is hydroxypropyl guar and is available from Rhône-Poulenc of Cranbury, NJ. | |

### Example 34

A face and scalp intense protection gel having the formulation in Table 20 below was prepared as follows. Jaguar C14S and Jaguar HP8C0S were mixed together and added to the deionized water while the deionized water was stirred. Solarcat and aloe gel were added to the solution and mixed. Tween 20, chamomille oil, and rosemary oil were mixed and added to the solution. Germazide MPB was added to the solution and mixed.

**Table 20**

| Ingredient | Percentage Weight (based upon 100% total weight of cream base) |
|---|---|
| Solarcat³¹ | 25.00 |
| Aloe Gel | 2.00 |
| Tween 20 | 0.15 |
| Chamomille Oil | 0.05 |
| Rosemary Oil | 0.05 |
| Germazide MPB | 1.50 |
| Deionized Water | 71.25 |

| | |
|---|---|
| ³¹ - Solarcat^{™} is a mixture of water, ethylhexyl methoxycinnamate, butyl methoxydibenzoylmethane, cyclomethicone, stearamidopropyl dimethylamine, stearamidopropyl dimethylamine stearate, and balm mint extract and is available from Collaborative Laboratories, Inc. of East Setauket, NY. | |

### Example 35

This example shows a cationic base containing oil dispersion sunscreens. A composition having the formulation in Table 21 below was prepared by mixing the ingredients either with a propeller or paddle blade mixer or with hand mixing with a spatula or other similar device.

**Table 21**

| Ingredient | Percentage Weight (based upon 100% total weight of cream base) |
|---|---|
| Cosmetic Base of Example 34 | 60.00 |
| Catezomes^{™} OMC³² | 10.00 |
| Germazide MPB | 1.50 |
| Deionized Water | 28.50 |

| | |
|---|---|
| ³² - Catezomes^{™} OMC is a mixture of ethylhexyl methoxycinnamate and stearamidopropyl dimethylamine stearate and is available from Collaborative Laboratories, Inc. of East Setauket, NY. | |

Many variations of the present invention will suggest themselves to those skilled in the art in light of the above, detailed description. All such obvious variations are within the full intended scope of the appended claims.

## Claims

1. A method of preparing a composition for topical, oral, nasal, anal, ophthalmic, or vaginal application, the method comprising mixing
(a) a premanufactured base composition comprising
(i) a rheology modifying agent, and
(ii) water; and
(b) at least one dispersion comprising suspended particles of a hydrophobic active agent, a hydrophobic adjuvant, or a combination thereof,
wherein the composition prepared comprises less than 3 per cent by weight of emulsifying surfactants based upon 100 per cent weight of total composition and the suspended particles have a diameter less than 1,000 nm.

2. The method of claim 1, wherein the composition comprises suspended particles having a diameter of from 50 to 500 nm.

3. The method of any one of claims 1-3, wherein the dispersion comprises oil.

4. The method of claim 3, wherein the oil is contained within oil droplets.

5. The method of claim 4, wherein the oil droplets have a diameter of from 250 to 500 nm.

6. The method of claim 4 or 5, wherein the oil droplets comprise one or more lipophilic materials.

7. The method of any one of claims 4-6, wherein the oil droplets have a charge as determined by zeta potential measurements.

8. The method of any one of claims 1-7, wherein the dispersion is prepared by high pressure mixing, high shear mixing, or a combination thereof.

9. The method of any one of claims 1-8, wherein the dispersion is prepared by ultra high shear mixing or microfluidization.

10. The method of any one of claims 1-9, wherein the mixing is performed by propeller mixing, paddle mixing, or sweep blade mixing.

11. The method of any one of claims 1-9, wherein the mixing is performed manually.

12. The method of any one of claims 1-11, wherein the mixing is performed without heating.

13. The method of any one of claims 1-11, wherein the mixing is performed at a temperature of from 15 to 30°C.

14. The method of claim 13, wherein the mixing is performed at a temperature of from 20 to 30°C.

15. A method for producing a customized composition of claim 1 for at least one of topical, oral, nasal, anal, ophthalmic, and vaginal application comprising the steps of:
providing at a first location (a) a premanufactured base composition comprising a rheology modifying agent and water and (b) a plurality of dispersions, the base composition being prepared at a second location, each dispersion comprising suspended particles of a hydrophobic active agent, a hydrophobic adjuvant, or a combination thereof;
receiving a customer order at the first location specifying properties of a desired composition;
selecting at least one of the plurality of dispersions in accordance with the customer order;
mixing a quantity of the base composition with quantities of the selected dispersions to form the customized composition, wherein the composition prepared comprises less than 3 per cent by weight of emulsifying surfactants based upon 100 per cent weight of total composition and the suspended particles have a diameter less than 1,000 nm.

16. The method of claim 15, wherein the mixing is performed at a temperature of from 20 to 30°C.

17. The method of claim 15 or 16, further comprising the steps of:
providing at least one aesthetic modifying agent at the first location; and
selecting at least one of the plurality of aesthetic modifying agents in accordance with the customer order,
the step of mixing comprising the step of mixing the quantity of base composition with the quantities of the selected dispersions and quantities of the selected aesthetic modifying agents to form the customized composition,
wherein the at least on aesthetic modifying agent is selected from the group consisting of:
- a mono, di, tri or polyalky ester of a di, tri or polyhydroxy compound;
- a mono, di, tri or polyalky ether of a di, tri or polyhydroxy compound;
- a compound having the formula CₙH₍₂ₙ₊₂₋ₘ₎ where n is an integer greater than or equal to 6 and m is 0 or an even integer no greater than n;
- a compound having the formula 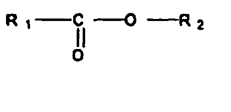 or the formula 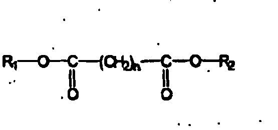
where R₁ is a saturated or unsaturated, linear, branched or cyclic C₁-C₂₄ alkyl; R₂ is hydrogen or a saturated or unsaturated, liner, branched or cyclic C₁-C₂₄ alkyl; and n is an integer from 0 to 20;
- polyalkylsiloxane, polyarylsitoxane, polyalkylarylsiloxane, polysiloxane gum and polyethersiloxane copolymer;
- a compound having has the formula 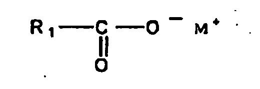
where R₁ is a saturated or unsaturated, linear, branched or cyclic alkyl having 2 to 24 carbon atoms; M⁽⁺⁾ is N⁺R₂R₃R₄R₅; R₂, R₃ and R₄ are hydrogen or a saturated or unsaturated, linear or branched alkyl or hydroxyalkyl having from 1 to 10 carbon atoms; and R₄ is a saturated or unsaturated, linear, branched or cyclic alkyl or substituted alkyl having 2 to 24 carbon atoms; and
- a polymer formed by polymerization of alkylene oxide monomers of the formula 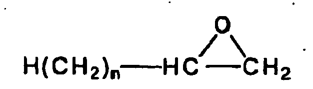
where n is an integer from 0 to 24.

18. The method of any one of claims 15-17, wherein the second location is remote from the first location.

19. The method of any one of claims 15-18, wherein the customer order originates at a location remote from the first location.

20. The method of any one of claims 15-18, wherein the customer order originates at the first location.

21. The method of any one of claims 15-20 further comprising the steps of:
indicating to the customer properties of the plurality of dispersions available at the first location; and
restricting the properties which can be specified by the customer to the indicated properties.

22. The method of any one of claims 15-21, wherein the selecting step further comprises determining if the selected dispersions and the base composition are compatible with each other.

23. The method of claim 22, further comprising the step of rejecting the order in response to a determination of incompatibility.

24. The method of claim 22 or 23, further comprising the step of proposing alternative selections to the customer in response to a determination of incompatibility.

25. The method of any one of claims 15-24, further comprising the step of proposing properties to the customer prior to the receiving step in accordance with environmental factors.

26. The method of any one of claims 15-25, wherein the environmental factors includes one or more of date, local humidity, and geographic region.

27. A method for producing a customized composition of claim 1 for at least one of topical, oral, nasal, anal, ophthalmic, and vaginal application comprising the steps of:
providing:
a) a premanufactured base composition comprising a rheology modifying agent,
b) a plurality of dispersions, each comprising suspended particles of a hydrophobic active agent, a hydrophobic adjuvant, or a combination thereof, and
c) at least one aesthetic modifying agent;
selecting at least one of the plurality of dispersions in accordance with a customer order; and
mixing at a temperature of from 20 to 30°C the base composition, the selected dispersions, and at least one selected aesthetic modifying agent to form a customized composition, wherein the composition prepared comprises less than 3 per cent by weight of emulsifying surfactants based upon 100 per cent weight of total composition and the suspended particles have a diameter less than 1,000 nm, and the at least on aesthetic modifying agent is selected from the group consisting of:
- a mono, di, tri or polyalky ester of a di, tri or polyhydroxy compound;
- a mono, di, tri or polyalky ether of a di, tri or polyhydroxy compound;
- a compound having the formula CₙH₍₂ₙ₊₂₋ₘ₎ where n is an integer greater than or equal to 6 and m is 0 or an even integer no greater than n;
- a compound having the formula 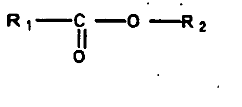 or the formula 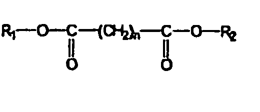
where R₁ is a saturated or unsaturated, linear, branched or cyclic C₁-C₂₄ alkyl; R₂ is hydrogen or a saturated or unsaturated, liner, branched or cyclic C₁-C₂₄ alkyl; and n is an integer from 0 to 20;
- polyalkylsiloxane, polyarylsiloxane, polyalkylarylsiloxane, polysiloxane gum and polyethersiloxane copolymer;
- a compound having has the formula 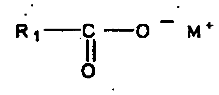
where R₁ is a saturated or unsaturated, linear, branched or cyclic alkyl having 2 to 24 carbon atoms; M⁽⁺⁾ is N⁺R₂R₃R₄R₅; R₂, R₃ and R₄ are hydrogen or a saturated or unsaturated, linear or branched alkyl or hydroxyalkyl having from 1 to 10 carbon atoms; and R₄ is a saturated or unsaturated, linear, branched or cyclic alkyl or substituted alkyl having 2 to 24 carbon atoms; and
- a polymer formed by polymerization of alkylene oxide monomers of the formula 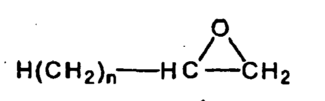
where n is an integer from 0 to 24.

28. The method of claim 27, wherein the at least one selected aesthetic modifying agent is selected in accordance with the customer order.

29. A method for producing a customized composition of claim 1 for at least one of topical, oral, nasal, anal, ophthalmic, and vaginal application comprising the steps of
preparing a base composition comprising a rheology modifying agent and water at a first location;
dehydrating the base composition;
transporting the base composition to a second location;
hydrating the base composition at the second location;
providing at a plurality of dispersions at the second location, each dispersion comprising suspended particles of a hydrophobic active agent, a hydrophobic adjuvant, or a combination thereof;
selecting at least one of the plurality of dispersions; and
mixing at a temperature of from 20 to 30°C a quantity of the base composition with quantities of the selected dispersions to form the customized composition.

30. The method of any one of claims 1-29, wherein the hydrophobic active agent is a sunscreen agent.

31. The method of any one of claims 1-29, wherein the hydrophobic active agent is a moisturizer.

32. Use of a system for producing a customized composition for at least one of topical, oral, nasal, anal, ophthalmic, and vaginal application according to the method of claim 1 comprising:
a computer server (40) configured to:
(a) receive a customer order for a customized composition from a client;
(b) generate a customized formulation in accordance with the customer order; and
(c) output the customized formulation for subsequent manufacture of the customized composition,
said system further comprising
(d) a dispensing unit (38), and
(e) a mixing unit (42)
wherein said server (40) is adapted to generate control signals to instruct said dispensing unit (38) to dispense determined quantities of compounds and pass them to said mixer (42) to produce said customised composition comprising
(A) a premanufactured base composition comprising
(i) a rheology modifying agent, and
(ii) water; and
(B) at least one dispersion comprising suspended particles of a hydrophobic active agent, a hydrophobic adjuvant, or a combination thereof,
wherein the composition prepared comprises less than 3 per cent by weight of emulsifying surfactants based upon 100 per cent weight of total composition and the suspended particles have a diameter less than 1,000nm.

33. A use according to claim 32, wherein the server is further configured to receive customer profile information, said customized formulation being generated in accordance with the customer order and the customer profile information.

34. A use according to claim 33, wherein the customer profile information is received from the client.

35. A use according to claim 33 or 34, wherein the customer profile information is received from a customer database.

36. A use according to any one of claims 32-35, wherein the computer system is further configured to receive an electronic image of the client and generate a user profile based thereon.

37. A use according to any one of claims 34-36, wherein the customer profile information is generated based upon the electronic image of the client and at least one biometric or physiological measurement.

38. A use according to any one of claims 32-37, wherein said customized formulation is generated in accordance with the customer order and environmental factors.

39. A use according to any one of claims 32-38, wherein the server is connected to the Internet.

40. A use according to any one of claims 32-39, wherein the server is remote from the customer.

## Patentansprüche

1. Verfahren zur Herstellung einer Zusammensetzung zur topischen, oralen, nasalen, analen, ophthalmischen oder vaginalen Anwendung, wobei das Verfahren das Mischen von
(a) einer vorgefertigten Grundzusammensetzung, umfassend
(i) ein Rheologie veränderndes Mittel; und
(ii) Wasser; und
(b) mindestens einer Dispersion, umfassend suspendierte Teilchen eines hydrophoben Wirkstoffes, eines hydrophoben Adjuvans oder einer Kombination davon,
umfasst,
wobei die hergestellte Zusammensetzung weniger als 3 Gew.-% emulgierender grenzflächenaktiver Mittel, bezogen auf 100 Gew.-% der gesamten Zusammensetzung, umfasst und die suspendierten Teilchen einen Durchmesser von weniger als 1000 nm aufweisen.

2. Verfahren nach Anspruch 1, wobei die Zusammensetzung suspendierte Teilchen, die einen Durchmesser von 50 bis 500 nm aufweisen, umfasst.

3. Verfahren nach einem der Ansprüche 1 bis 3, wobei die Dispersion Öl umfasst.

4. Verfahren nach Anspruch 3, wobei das Öl in Öltropfen enthalten ist.

5. Verfahren nach Anspruch 4, wobei die Öltropfen einen Durchmesser von 250 bis 500 nm aufweisen.

6. Verfahren nach Anspruch 4 oder 5, wobei die Öltropfen ein oder mehrere lipophile Materialien umfassen.

7. Verfahren nach einem der Ansprüche 4 bis 6, wobei die Öltropfen eine durch Zeta-Potential-Messungen bestimmte Ladung aufweisen.

8. Verfahren nach einem der Ansprüche 1 bis 7, wobei die Dispersion durch Hochdruckmischen, Mischen unter hoher Scherkraft oder eine Kombination davon hergestellt wird.

9. Verfahren nach einem der Ansprüche 1 bis 8, wobei die Dispersion durch Mischen unter ultrahoher Scherkraft oder Mikrofluidisierung hergestellt wird.

10. Verfahren nach einem der Ansprüche 1 bis 9, wobei das Mischen durch Propellerrühren, Schaufelrühren oder Schaufelkehrrühren (engl. sweep blade mixing) durchgeführt wird.

11. Verfahren nach einem der Ansprüche 1 bis 9, wobei das Mischen per Hand durchgeführt wird.

12. Verfahren nach einem der Ansprüche 1 bis 11, wobei das Mischen ohne Erwärmen durchgeführt wird.

13. Verfahren nach einem der Ansprüche 1 bis 11, wobei das Mischen bei einer Temperatur von 15 bis 30°C durchgeführt wird.

14. Verfahren nach Anspruch 13, wobei das Mischen bei einer Temperatur von 20 bis 30°C durchgeführt wird.

15. Verfahren zur Herstellung einer kundenspezifischen Zusammensetzung nach Anspruch 1 für mindestens eine aus einer topischen, oralen, nasalen, analen, ophthalmischen und vaginalen Anwendung, umfassend die Schritte:
Bereitstellen an einer ersten Stelle (a) einer vorgefertigten Grundzusammensetzung, umfassend ein Rheologie veränderndes Mittel und Wasser und (b) einer Mehrzahl von Dispersionen, wobei die Grundzusammensetzung an einer zweiten Stelle hergestellt wird, wobei jede Dispersion suspendierte Teilchen eines hydrophoben Wirkstoffes, eines hydrophoben Adjuvans oder einer Kombination davon umfasst;
Empfangen eines Kundenauftrags, der die Eigenschaften einer gewünschten Zusammensetzung festlegt, an der ersten Stelle;
Auswählen mindestens einer aus der Mehrzahl von Dispersionen gemäß dem Kundenauftrag;
Mischen einer Menge der Grundzusammensetzung mit Mengen der ausgewählten Dispersionen, um die kundenspezifische Zusammensetzung zu bilden, wobei die hergestellte Zusammensetzung weniger als 3 Gew.-% emulgierender grenzflächenaktiver Mittel, bezogen auf 100 Gew.-% der gesamten Zusammensetzung, umfasst und die suspendierten Teilchen einen Durchmesser von weniger als 1000 nm aufweisen.

16. Verfahren nach Anspruch 15, wobei das Mischen bei einer Temperatur von 20 bis 30°C durchgeführt wird.

17. Verfahren nach Anspruch 15 oder 16, weiterhin umfassend die Schritte:
Bereitstellen mindestens eines Aussehen verändernden Mittels an der ersten Stelle; und
Auswählen mindestens eines aus der Mehrzahl der Aussehen verändernden Mittel gemäß dem Kundenauftrag;
wobei der Schritt des Mischens den Schritt des Mischens der Menge der Grundzusammensetzung mit den Mengen der ausgewählten Dispersionen und Mengen der ausgewählten Aussehen verändernden Mittel umfasst, um die kundenspezifische Zusammensetzung zu bilden, wobei das mindestens eine Aussehen verändernde Mittel ausgewählt ist aus:
- einem Mono-, Di-, Tri- oder Polyalkylester einer Di-, Tri- oder Polyhydroxyverbindung
- einem Mono-, Di-, Tri- oder Polyalkylether einer Di-, Tri- oder Polyhydroxyverbindung
- einer Verbindung der Formel CₙH(₂ₙ₊₂₋ₘ), wobei n eine ganze Zahl größer als oder gleich 6 ist und m 0 oder eine gerade ganze Zahl nicht größer als n ist;
- einer Verbindung der Formel 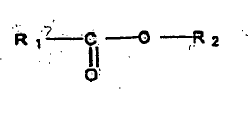 oder der Formel 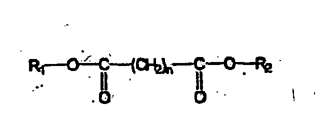
wobei R₁ ein gesättigter oder ungesättigter, geradkettiger, verzweigter oder cyclischer C₁-C₂₄-Alkylrest ist; R₂ Wasserstoff oder ein gesättigter oder ungesättigter, geradkettiger, verzweigter oder cyclischer C₁-C₂₄-Alkylrest ist; und n eine ganze Zahl von 0 bis 20 ist;
- Polyalkylsiloxan, Polyarylsiloxan, Polyalkylarylsiloxan, Polysiloxangummi und Polyethersiloxancopolymer;
- einer Verbindung der Formel 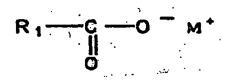
wobei R₁ ein gesättigter oder ungesättigter, geradkettiger, verzweigter oder cyclischer Alkylrest mit 2 bis 24 Kohlenstoffatomen ist; M(⁺) für N⁺R₂R₃R₄R₅ steht; R₂, R₃ und R₄ Wasserstoff oder ein gesättigter oder ungesättigter, geradkettiger oder verzweigter Alkyl- oder Hydroxyalkylrest mit 1 bis 10 Kohlenstoffatomen sind; und R₄ ein gesättigter oder ungesättigter, geradkettiger, verzweigter oder cyclischer Alkylrest oder substituierter Alkylrest mit 2 bis 24 Kohlenstoffatomen ist; und
- einem durch Polymerisation von Alkylenoxidmonomeren der Formel 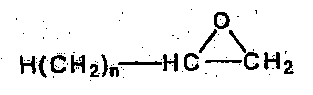
wobei n eine ganze Zahl von 0 bis 24 ist, erhaltenen Polymer.

18. Verfahren nach einem der Ansprüche 15 bis 17, wobei die zweite Stelle von der ersten Stelle entfernt ist.

19. Verfahren nach einem der Ansprüche 15 bis 18, wobei der Kundenauftrag von einer Stelle ausgeht, die von der ersten Stelle entfernt ist.

20. Verfahren nach einem der Ansprüche 15 bis 18, wobei der Kundenauftrag von der ersten Stelle ausgeht.

21. Verfahren nach einem der Ansprüche 15 bis 20, weiterhin umfassend die Schritte:
Bekanntgeben der Eigenschaften der Mehrzahl an Dispersionen, die an der ersten Stelle verfügbar sind, an den Kunden; und
Beschränken der Eigenschaften, die durch den Kunden bestimmt werden können, auf die angegebenen Eigenschaften.

22. Verfahren nach einem der Ansprüche 15 bis 21, wobei der Schritt des Auswählens weiterhin das Bestimmen, ob die ausgewählten Dispersionen und die Grundzusammensetzung miteinander kompatibel sind, umfasst.

23. Verfahren nach Anspruch 22, weiterhin umfassend den Schritt des Ablehnens des Auftrags als Reaktion auf eine Bestimmung von Inkompatibilität.

24. Verfahren nach Anspruch 22 oder 23, weiterhin umfassend den Schritt, dass dem Kunden alternative Auswahlmöglichkeiten als Reaktion auf eine Bestimmung von Inkompatibilität vorgeschlagen werden.

25. Verfahren nach einem der Ansprüche 15 bis 24, weiterhin umfassend den Schritt, dass dem Kunden vor dem Schritt des Empfangens Eigenschaften passend zu den jeweiligen Umgebungsfaktoren vorgeschlagen werden.

26. Verfahren nach einem der Ansprüche 15 bis 25, wobei die Umgebungsfaktoren einen oder mehrere Faktoren aus Datum, örtlicher Feuchtigkeit und geographischer Region einschließen.

27. Verfahren zur Herstellung einer kundenspezifischen Zusammensetzung nach Anspruch 1 für mindestens eine aus einer topischen, oralen, nasalen, analen, ophthalmischen und vaginalen Anwendung, umfassend die Schritte:
Bereitstellen
a) einer vorgefertigten Grundzusammensetzung, umfassend ein Rheologie veränderndes Mittel,
b) einer Mehrzahl von Dispersionen, wobei jede suspendierte Teilchen eines hydrophoben Wirkstoffes, eines hydrophoben Adjuvans oder einer Kombination davon umfasst, und
c) mindestens eines Aussehen verändernden Mittels;
Auswählen mindestens einer aus der Mehrzahl der Dispersionen gemäß einem Kundenauftrag; und
Mischen der Grundzusammensetzung, der ausgewählten Dispersionen und des mindestens eines Aussehen verändernden Mittels bei einer Temperatur von 20 bis 30°C, um eine kundenspezifische Zusammensetzung zu bilden, wobei die hergestellte Zusammensetzung weniger als 3 Gew.-% emulgierender grenzflächenaktiver Mittel, bezogen auf 100 Gew.-% der gesamten Zusammensetzung, umfasst und die suspendierten Teilchen einen Durchmesser von weniger als 1000 nm aufweisen, und das mindestens eine Aussehen verändernde Mittel ausgewählt ist aus:
- einem Mono-, Di-, Tri- oder Polyalkylester einer Di-, Tri- oder Polyhydroxyverbindung
- einem Mono-, Di-, Tri- oder Polyalkylether einer Di-, Tri- oder Polyhydroxyverbindung
- einer Verbindung der Formel CₙH₍₂ₙ₊₂₋ₘ₎, wobei n eine ganze Zahl größer als oder gleich 6 ist und m 0 oder eine gerade ganze Zahl nicht größer als n ist;
- einer Verbindung der Formel 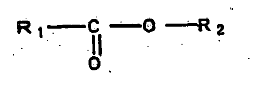 oder der Formel 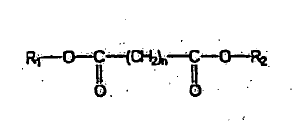
wobei R₁ ein gesättigter oder ungesättigter, geradkettiger, verzweigter oder cyclischer C₁-C₂₄-Alkylrest ist; R₂ Wasserstoff oder ein gesättigter oder ungesättigter, geradkettiger, verzweigter oder cyclischer C₁-C₂₄-Alkylrest ist; und n eine ganze Zahl von 0 bis 20 ist;
- Polyalkylsiloxan, Polyarylsiloxan, Polyalkylarylsiloxan, Polysiloxangummi und Polyethersiloxancopolymer;
- einer Verbindung der Formel 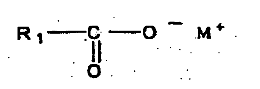
wobei R₁ ein gesättigter oder ungesättigter, geradkettiger, verzweigter oder cyclischer Alkylrest mit 2 bis 24 Kohlenstoffatomen ist; M⁽⁺⁾ für N⁺R₂R₃R₄R₅ steht; R₂, R₃ und R₄ Wasserstoff oder ein gesättigter oder ungesättigter, geradkettiger oder verzweigter Alkyl- oder Hydroxyalkylrest mit 1 bis 10 Kohlenstoffatomen sind; und R₄ ein gesättigter oder ungesättigter, geradkettiger, verzweigter oder cyclischer Alkylrest oder substituierter Alkylrest mit 2 bis 24 Kohlenstoffatomen ist; und
- einem durch Polymerisation von Alkylenoxidmonomeren der Formel 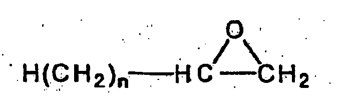
wobei n eine ganze Zahl von 0 bis 24 ist, erhaltenen Polymer.

28. Verfahren nach Anspruch 27, wobei das mindestens eine ausgewählte Aussehen verändernde Mittel gemäß dem Kundenauftrag ausgewählt ist.

29. Verfahren zur Herstellung einer kundenspezifischen Zusammensetzung nach Anspruch 1 für mindestens eine aus einer topischen, oralen, nasalen, analen, ophthalmischen und vaginalen Anwendung, umfassend die Schritte:
Herstellen einer Grundzusammensetzung, umfassend ein Rheologie veränderndes Mittel und Wasser an einer ersten Stelle;
Dehydratisieren der Grundzusammensetzung;
Transportieren der Grundzusammensetzung an eine zweite Stelle;
Hydratisieren der Grundzusammensetzung an der zweiten Stelle;
Bereitstellen einer Mehrzahl von Dispersionen an der zweiten Stelle, wobei jede Dispersion suspendierte Teilchen eines hydrophoben Wirkstoffes, eines hydrophoben Adjuvans oder einer Kombination davon umfasst;
Auswählen mindestens einer aus der Mehrzahl von Dispersionen; und
Mischen einer Menge der Grundzusammensetzung mit Mengen der ausgewählten Dispersionen bei einer Temperatur von 20 bis 30°C, um die kundenspezifische Zusammensetzung zu bilden.

30. Verfahren nach einem der Ansprüche 1 bis 29, wobei der hydrophobe Wirkstoff ein Sonnenschutzmittel ist.

31. Verfahren nach einem der Ansprüche 1 bis 29, wobei der hydrophobe Wirkstoff ein Feuchtigkeitsmittel ist.

32. Verwendung eines Systems zur Herstellung einer kundenspezifischen Zusammensetzung für mindestens eine aus einer topischen, oralen, nasalen, analen, ophthalmischen und vaginalen Anwendung gemäß dem Verfahren nach Anspruch 1, umfassend:
einen Computerserver (40), der konfiguriert ist, um
a) einen Kundenauftrag für eine kundenspezifische Zusammensetzung von einem Kunden zu empfangen;
b) eine kundenspezifische Formulierung gemäß dem Kundenauftrag herzustellen; und
c) die kundenspezifische Formulierung zur nachfolgenden Herstellung der kundenspezifischen Zusammensetzung auszugeben,
wobei das System weiterhin umfasst:
d) eine Dispensiereinheit (38), und
e) eine Mischereinheit (42)
wobei der Server (40) so angepasst ist, Steuersignale zu erzeugen, um die Dispensiereinheit (38) anzuweisen, bestimmte Mengen von Verbindungen zu dispensieren und sie an den Mischer (42) weiter zu geben, um die kundenspezifische Zusammensetzung herzustellen, umfassend
(A) eine vorgefertigte Grundzusammensetzung, umfassend
(i) ein Rheologie veränderndes Mittel; und
(ii) Wasser; und
(B) mindestens eine Dispersion, die suspendierte Teilchen eines hydrophoben Wirkstoffes, eines hydrophoben Adjuvans oder einer Kombination davon, umfasst,
wobei die hergestellte Zusammensetzung weniger als 3 Gew.-% emulgierender grenzflächenaktiver Mittel, bezogen auf 100 Gew.-% der gesamten Zusammensetzung, umfasst und die suspendierten Teilchen einen Durchmesser von weniger als 1000 nm aufweisen.

33. Verwendung nach Anspruch 32, wobei der Server weiterhin konfiguriert ist, eine Kundenprofilinformation zu empfangen, wobei die kundenspezifische Formulierung gemäß dem Kundenauftrag und der Kundenprofilinformation erstellt wird.

34. Verwendung nach Anspruch 33, wobei die Kundenprofilinformation vom Kunden erhalten wird.

35. Verwendung nach Anspruch 33 oder 34, wobei die Kundenprofilinformation von einer Kundendatenbank erhalten wird.

36. Verwendung nach einem der Ansprüche 32 bis 35, wobei das Computersystem weiterhin konfiguriert ist, ein elektronisches Bild des Kunden zu empfangen und darauf basierend ein Benutzerprofil zu erstellen.

37. Verwendung nach einem der Ansprüche 34 bis 36, wobei die Kundenprofilinformation basierend auf dem elektronischen Bild des Kunden und mindestens einer biometrischen oder physiologischen Messung erstellt wird.

38. Verwendung nach einem der Ansprüche 32 bis 37, wobei die kundenspezifische Formulierung gemäß dem Kundenauftrag und Umgebungsfaktoren hergestellt wird.

39. Verwendung nach einem der Ansprüche 32 bis 38, wobei der Server mit dem Internet verbunden ist.

40. Verwendung nach einem der Ansprüche 32 bis 39, wobei der Server vom Kunden entfernt ist.

## Revendications

1. Procédé de préparation d'une composition pour une application topique, orale, nasale, anale, ophtalmique ou vaginale, le procédé comprenant le mélange
(a) d'une composition de base pré-manufacturée comprenant
(i) un agent de modification de rhéologie, et
(ii) de l'eau ; et
(b) au moins une dispersion comprenant des particules en suspension d'agent actif hydrophobe, un adjuvant hydrophobe ou une combinaison de ceux-ci,
dans lequel la composition préparée comprend moins de 3 % en poids d'agents tensioactifs émulsifiants sur une base de 100 % en poids de la composition totale et les particules en suspension présentent un diamètre inférieur à 1 000 nm.

2. Procédé selon la revendication 1, dans lequel la composition comprend des particules en suspension ayant un diamètre de 50 à 500 nm.

3. Procédé selon l'une quelconque des revendications 1 à 3, dans lequel la dispersion comprend de l'huile.

4. Procédé selon la revendication 3, dans lequel l'huile est contenue à l'intérieur de gouttelettes d'huile.

5. Procédé selon la revendication 4, dans lequel les gouttelettes d'huile présentent un diamètre de 250 à 500 nm.

6. Procédé selon la revendication 4 ou la revendication 5, dans lequel les gouttelettes d'huile comprennent un ou plusieurs matériaux lipophiles.

7. Procédé selon l'une quelconque des revendications 4 à 6, dans lequel les gouttelettes d'huile présentent une charge telle que déterminée par des mesures de potentiel zêta.

8. Procédé selon l'une quelconque des revendications 1 à 7, dans lequel la dispersion est préparée par un mélange à haute pression, un mélange à cisaillement élevé ou une combinaison de ceux-ci.

9. Procédé selon l'une quelconque des revendications 1 à 8, dans lequel la dispersion est préparée par un mélange à cisaillement ultra élevé ou par une micro-fluidisation.

10. Procédé selon l'une quelconque des revendications 1 à 9, dans lequel le mélange est réalisé par un mélangeur à hélices, à palettes ou à lames.

11. Procédé selon l'une quelconque des revendications 1 à 9, dans lequel le mélange est réalisé manuellement.

12. Procédé selon l'une quelconque des revendications 1 à 11, dans lequel le mélange est réalisé sans chauffage.

13. Procédé selon l'une quelconque des revendications 1 à 11, dans lequel le mélange est réalisé à une température de 15 à 30°C.

14. Procédé selon la revendication 13, dans lequel le mélange est réalisé à une température de 20 à 30°C.

15. Procédé de production d'une composition à la demande selon la revendication 1 pour au moins une application parmi une application topique, orale, nasale, anale, ophtalmique et vaginale comprenant les étapes de :
détermination d'un premier lieu (a) d'une composition de base pré-manufacturée comprenant un agent de modification de rhéologie et de l'eau et (b) d'une pluralité de dispersions, la composition de base étant préparée dans un second lieu, chaque dispersion comprenant des particules en suspension d'un agent actif hydrophobe, un adjuvant hydrophobe ou une combinaison de ceux-ci ;
réception d'une commande d'un client à la première position spécifiant des propriétés d'une composition souhaitée ;
sélection d'au moins une pluralité de dispersions conformément à la commande du client ;
mélange d'une quantité de la composition de base avec des quantités de dispersions choisies pour former la composition sur demande, dans lequel la composition préparée comprend moins de 3 % en poids d'agents tensioactifs émulsifiants sur base de 100 % en poids de la composition totale et les particules en suspension présentent un diamètre inférieur à 1 000 nm.

16. Procédé selon la revendication 15, dans lequel le mélange est réalisé à une température de 20 à 30°C.

17. Procédé selon la revendication 15 ou la revendication 16, comprenant en outre les étapes de :
fournir au moins un agent de modification esthétique au premier lieu ; et
sélection d'au moins un parmi la pluralité des agents de modification esthétique conformément à la commande du client ;
l'étape de mélange comprenant l'étape de mélange de la quantité de la composition de base avec les quantités des dispersions choisies et des quantités des agents de modification esthétique choisis pour former la composition à la demande, dans laquelle le au moins un agent de modification esthétique est choisi dans le groupe constitué de :
- un mono-, di-, tri- ou polyalkylester d'un composé di, tri ou polyhydroxy ;
- un mono-, di-, tri- ou polyalkyléther d'un composé di, tri ou polyhydroxy ;
- un composé ayant la formule CₙH₍₂ₙ₊₂₋ₘ₎ dans laquelle n est un entier supérieur ou égal à 6 et m est 0 ou un entier pair non supérieur à n ;
- un composé ayant la formule 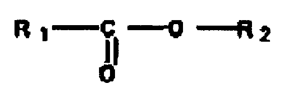
ou la formule 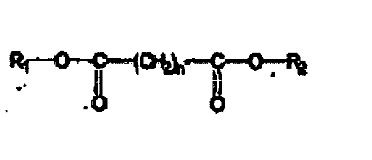 dans laquelle R₁ est un groupe alkyle saturé ou insaturée, linéaire, ramifié ou cyclique en C₁ à C₂₄; R₂ est un atome d'hydrogène ou un groupe alkyle saturé ou insaturé, linéaire, ramifié ou cyclique en C₁ à C₂₄ ; et n est un entier de 0 à 20 ;
- une gomme de polyalkylsiloxane, de polyarylsiloxane, de polyalkylarylsiloxane, de polysiloxane et un copolymère de polyéthersiloxane ;
- un composé ayant la formule 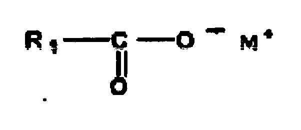
dans laquelle R₁ est un groupe alkyle saturé ou insaturé, linéaire, ramifié ou cyclique ayant de 2 à 24 atomes de carbone ; M⁽⁺⁾ est N⁺R₂R₃R₄R₅ ; R₂, R₃ et R₄ sont un atome d'hydrogène ou un groupe alkyle ou hydroxyalkyle, saturé ou insaturé, linéaire ou ramifié ayant de 1 à 10 atomes de carbone ; et R₄ est un groupe alkyle ou alkyle substitué, saturé ou insaturé, linéaire, ramifié ou cyclique ayant de 2 à 24 atomes de carbone ; et
- un polymère formé par la polymérisation de monomères d'oxyde d'alkylène de la formule 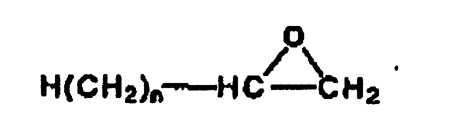
dans laquelle n est un entier de 0 à 24.

18. Procédé selon l'une quelconque des revendications 15 à 17, dans lequel le second lieu est éloigné de premier lieu.

19. Procédé selon l'une quelconque des revendications 15 à 18, dans lequel la commande du client provient d'un lieu éloigné du premier lieu.

20. Procédé selon l'une quelconque des revendications 15 à 18, dans lequel la commande du client provient du premier lieu.

21. Procédé selon l'une quelconque des revendications 15 à 20 comprenant en outre les étapes de :
indication au client des propriétés de la pluralité des dispersions disponibles au premier lieu ; et
restriction des propriétés qui peuvent être spécifiées par le client aux propriétés indiquées.

22. Procédé selon l'une quelconque des revendications 15 à 21, dans lequel l'étape de sélection comprend en outre l'étape consistant à déterminer si les dispersions choisies et la composition de base sont compatibles les unes avec l'autre.

23. Procédé selon la revendication 22, comprenant en outre l'étape de rejet de la commande en réponse à une détermination d'incompatibilité.

24. Procédé selon la revendication 22 ou la revendication 23, comprenant en outre l'étape de proposition de sélections alternatives au client en réponse à une détermination d'incompatibilité.

25. Procédé selon l'une quelconque des revendications 15 à 24, comprenant en outre l'étape de proposition de propriétés au client avant l'étape de réception conformément aux facteurs environnementaux.

26. Procédé selon l'une quelconque des revendications 15 à 25, dans lequel les facteurs environnementaux comprennent un ou plusieurs parmi la date, l'humidité locale et la région géographique.

27. Procédé de production d'une composition sur demande selon la revendication 1 pour au moins une application parmi une application topique, orale, nasale, anale, ophtalmique et vaginale comprenant les étapes consistant à :
fournir
a) une composition de base pré-manufacturée comprenant un agent de modification de rhéologie,
b) une pluralité de dispersions, chaque dispersion comprenant des particules en suspension d'un agent actif hydrophobe, un adjuvant hydrophobe ou une combinaison de ceux-ci, et
c) au moins un agent de modification esthétique ;
sélectionner au moins une parmi la pluralité de dispersions conformément à une commande d'un client, et
mélanger à une température allant de 20 à 30°C, la composition de base, les dispersions choisies et au moins un agent de modification esthétique pour former une composition sur demande, dans laquelle la composition préparée comprend moins de 3 % en poids d'agents tensioactifs émulsifiants sur une base de 100% en poids de la composition totale et les particules en suspension présentent un diamètre inférieur à 1 000 nm et le au moins un agent de modification esthétique est choisi dans le groupe comprenant :
- un mono-, di-, tri- ou polyalkylester d'un composé di, tri ou polyhydroxy ;
- un mono-, di-, tri- ou polyalkyléther d'un composé di, tri ou polyhydroxy ;
- un composé ayant la formule CₙH₍₂ₙ₊₂₋ₘ₎ dans laquelle n est un entier supérieur ou égal à 6 et m est 0 ou un entier pair non supérieur à n ;
- un composé ayant la formule 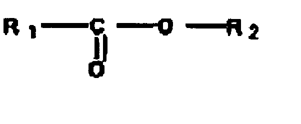
ou la formule 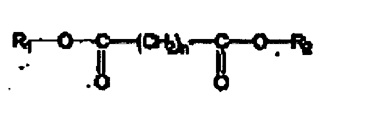 dans laquelle R₁ est un groupe alkyle saturé ou insaturé, linéaire, ramifié ou cyclique en C₁ à C₂₄ ; R₂ est un atome d'hydrogène ou un groupe alkyle saturé ou insaturé, linéaire, ramifié ou cyclique en C₁ à C₂₄ ; et n est un entier de 0 à 20 ;
- une gomme de polyalkylsiloxane, de polyarylsiloxane, de polyalkylarylsiloxane, de polysiloxane et un copolymère de polyéthersiloxane ;
- un composé ayant la formule 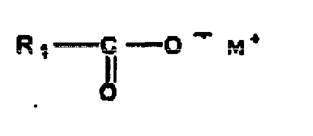
dans laquelle R₁ est un groupe alkyle saturé ou insaturé, linéaire, ramifié ou cyclique ayant de 2 à 24 atomes de carbone ; M(⁺) est N⁺R₂R₃R₄R₅ ; R₂, R₃ et R₄ sont un atome d'hydrogène ou un groupe alkyle ou hydroxyalkyle, saturé ou insaturé, linéaire ou ramifié ayant de 1 à 10 atomes de carbone ; et R₄ est un groupe alkyle ou alkyle substitué, saturé ou insaturé, linéaire, ramifié ou cyclique ayant de 2 à 24 atomes de carbone ; et
- un polymère formé par la polymérisation de monomères d'oxyde d'alkylène de la formule 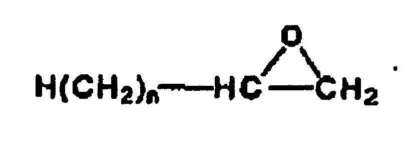
dans laquelle n est un entier de 0 à 24.

28. Procédé selon la revendication 27, dans lequel le au moins un agent de modification esthétique choisi est choisi conformément à la commande du client.

29. Procédé de production d'une composition sur demande selon la revendication 1 pour au moins une application parmi une application topique, orale, nasale, anale, ophtalmique et vaginale comprenant les étapes de :
préparation d'une composition de base comprenant un agent de modification de rhéologie et de l'eau en un premier lieu ;
déshydratation de la composition de base ;
transport de la composition de base vers un second lieu ;
hydratation de la composition de base dans un second lieu;
fournir une pluralité de dispersions dans le second lieu, chaque dispersion comprenant des particules en suspension d'un agent actif hydrophobe, un adjuvant hydrophobe ou une combinaison de ceux-ci ;
choix d'au moins une parmi la pluralité de dispersions ; et
mélange à une température allant de 20 à 30°C d'une quantité de la composition de base avec des quantités de dispersions choisies pour former la composition sur demande.

30. Procédé selon l'une quelconque des revendications 1 à 29, dans lequel l'agent actif hydrophobe est un agent écran solaire.

31. Procédé selon l'une quelconque des revendications 1 à 29, dans lequel l'agent actif hydrophobe est un agent hydratant.

32. Utilisation d'un système de production d'une composition sur demande pour au moins une application parmi une application topique, orale, nasale, anale, ophtalmique et vaginale selon le procédé de la revendication 1 comprenant :
un serveur informatique (40) configuré de manière à :
(a) recevoir une commande d'un client pour une composition sur demande d'un client ;
(b) générer une formulation sur demande conformément à la commande du client ; et
(c) sortir la formulation sur demande pour la production subséquente de la composition sur demande,
ledit système comprenant en outre
(d) une unité de mise en suspension (38), et
(e) une unité de mélange (42)
dans lequel ledit serveur (40) est adapté de manière à produire des signaux de contrôle pour donner à ladite unité de distribution (38) des instructions pour qu'elle détermine les quantités de composés et les envoyer audit mélangeur (42) pour produire ladite composition sur demande comprenant
(A) une composition de base pré-manufacturée comprenant
(i) un agent de modification de rhéologie, et
(ii) de l'eau ; et
(B) au moins une dispersion comprenant des particules en suspension d'un agent actif hydrophobe, un adjuvant hydrophobe ou une combinaison de ceux-ci, dans laquelle la composition préparée comprend moins de 3 % en poids d'agents tensioactifs émulsifiants sur la base de 100 % en poids de la composition totale et les particules en suspension présentent un diamètre inférieur à 1 000 nm.

33. Utilisation selon la revendication 32, dans laquelle le serveur est configuré en outre pour recevoir des informations de profil du client, ladite formulation sur demande étant produite conformément à la commande du client et les informations de profil du client.

34. Utilisation selon la revendication 33, dans lequel les informations de profil du client sont reçues du client.

35. Utilisation selon la revendication 33 ou la revendication 34, dans laquelle les informations de profil du client sont reçues d'une base de données du client.

36. Utilisation selon l'une quelconque des revendications 32 à 35, dans laquelle le système informatique est en outre configuré pour recevoir une image électronique du client et générer un profil d'utilisateur basé sur celle-ci.

37. Utilisation selon l'une quelconque des revendications 34 à 36, dans laquelle les informations de profil du client sont produites sur base de l'image électronique du client et au moins une mesure biométrique ou physiologique.

38. Utilisation selon l'une quelconque des revendications 32 à 37, dans laquelle ladite formulation sur demande est produite conformément à la commande du client et des facteurs environnementaux.

39. Utilisation selon l'une quelconque des revendications 32 à 38, dans laquelle le serveur est connecté à Internet.

40. Utilisation selon l'une quelconque des revendications 32 à 39, dans laquelle le serveur est éloigné du client.
